# EUROPEAN PATENT SPECIFICATION

(11) **EP 3 768 324 B1**
(45) Date of publication and mention of the grant of the patent: **26.11.2025**
(21) Application number: 19711136.2
(22) Date of filing: 21.03.2019
(51) Int. Cl.: A61K 47/68

(54) **METHODS FOR MODULATING INNATE LYMPHOID CELL ACTIVITY, ANTIBODY DRUG CONJUGATES AND USES IN THERAPY**
VERFAHREN ZUR MODULIERUNG DER AKTIVITÄT INNATER LYMPHOIDER ZELLEN, ANTIKÖRPER-ARZNEIMITTELKONJUGATE UND VERWENDUNGEN IN DER THERAPIE
PROCÉDÉS DE MODULATION DE L'ACTIVITÉ DES CELLULES LYMPHOÏDES INNÉES, CONJUGUÉS ANTICORPS-MÉDICAMENT ET LEURS UTILISATIONS EN THÉRAPIE

(30) Priority: 22.03.2018 EP 18305319
(43) Date of publication of application: 27.01.2021
(73) Proprietor: INSERM (Institut National de la Santé et de la Recherche Médicale), 75013 Paris (FR); CENTRE NATIONAL DE LA RECHERCHE SCIENTIFIQUE -CNRS-, 75016 Paris (FR); Université d'Aix Marseille, 13284 Marseille Cedex 07 (FR)
(72) Inventor: UGOLINI, Sophie, 13288 Marseille Cedex 09 (FR); QUATRINI, Linda, 13288 Marseille - Cedex 09 (FR)
(74) Representative: Inserm Transfert
(86) International application number: PCT/EP2019/057101
(87) International publication number: WO 2019/180150

(56) References cited:
- WO-A2-2017/062271
- US-A1- 2011 269 728
- US-A1- 2017 044 259
- ALSTON CHRISTINE I ET AL: "Reduced frequency of murine cytomegalovirus retinitis in C57BL/6 mice correlates with low levels of suppressor of cytokine signaling (SOCS)1 and SOCS3 expression within the eye during corticosteroid-induced immunosuppression", CYTOKINE, ACADEMIC PRESS LTD, PHILADELPHIA, PA, US, vol. 97, 28 May 2017 (2017-05-28), pages 38 - 41, XP085123391, ISSN: 1043-4666, DOI: 10.1016/J.CYTO.2017.05.021
- EDDY JUSTIN L. ET AL: "Glucocorticoids regulate natural killer cell function epigenetically", CELLULAR IMMUNOLOGY, vol. 290, no. 1, 1 July 2014 (2014-07-01), US, pages 120 - 130, XP0055963944, ISSN: 0008-8749, Retrieved from the Internet <URL:https://www.ncbi.nlm.nih.gov/pmc/articles/PMC4128186/pdf/nihms610909.pdf> DOI: 10.1016/j.cellimm.2014.05.013
- NORIKO OGASAWARA ET AL: "IL-10, TGF-[beta], and glucocorticoid prevent the production of type 2 cytokines in human group 2 innate lymphoid cells", JOURNAL OF ALLERGY AND CLINICAL IMMUNOLOGY, vol. 141, no. 3, 23 October 2017 (2017-10-23), AMSTERDAM, NL, pages 1147 - 1151.e8, XP055512483, ISSN: 0091-6749, DOI: 10.1016/j.jaci.2017.09.025
- LIU SUCAI ET AL: "Glucocorticoids Act both Antagonistically and Protagonistically on Type 2 Innate Lymphoid Cells (ILC2s) Depending upon the Stage of Development and the Cytokine Milieu", JOURNAL OF ALLERGY AND CLINICAL IMMUNOLOGY, vol. 139, no. 2, 28 February 2017 (2017-02-28), XP029935078, ISSN: 0091-6749, DOI: 10.1016/J.JACI.2016.12.632

## Description

### FIELD OF THE INVENTION:

The present invention relates to methods for modulating innate lymphoid cell activity, antibody drug conjugates and uses in therapy.

### BACKGROUND OF THE INVENTION:

The immune system protects the host organism against infectious diseases, principally by eradicating infectious agents. However, pathogen elimination is frequently accompanied by collateral tissue damage and inflammation, which may have highly deleterious effects on host fitness. An ability to modulate the immune response and to tolerate, at least partly, the presence of pathogens, is, thus, also essential for the global defense strategy of the organism (Medzhitov, et al. Disease tolerance as a defense strategy. Science 335, 936-941, (2012)). Neuroendocrine-immune interactions play an important role in these regulatory processes (Irwin, M. R. & Cole, S. W. Reciprocal regulation of the neural and innate immune systems. Nat Rev Immunol 11, 625-632 (2011)), but the mechanisms involved remain unclear. The hypothalamic-pituitary-adrenal (HPA) axis is activated upon infection, to produce endogenous glucocorticoids, steroid hormones essential for host protection against the deleterious effects of excessive inflammation (Webster, J. I., et al. Neuroendocrine regulation of immunity. Annu Rev Immunol 20, 125-163, (2002).). The anti-inflammatory properties of glucocorticoids are widely used in clinical practice. However, these hormones have pleiotropic actions on multiple cellular targets, and the cell lineage-specific molecular mechanisms underlying their immunoregulatory function are poorly understood (Cain, D. W. & Cidlowski, J. A. Immune regulation by glucocorticoids. Nat Rev Immunol 17, 233-247, (2017)). The tissue-specific or cells-specific use of glucocorticoid for treating conditions appears to be novel promising drugs. The international patent application WO2017/062271 describes antibody-drug conjugates comprising an antibody that target the human CD25, human CD70, human CD74 protein, or human CD 163 protein conjugated to an anti-inflammatory therapeutic agent. However, there is still a great need for providing efficient glucocorticoids therapeutic strategies targeting specific tissue or specific cells depending on the condition to treat.

### SUMMARY OF THE INVENTION

The present invention relates to methods for modulating innate lymphoid cell activity, antibody drug conjugates and uses in therapy, as defined by the claim.

### DETAILED DESCRIPTION OF THE INVENTION:

The inventors show here in a model of murine cytomegalovirus (MCMV) infection, that glucocorticoid receptor (GR) expression in NCR1+ innate lymphoid cells (ILCs) plays an essential early role in regulating host protection against inflammation-induced tissue damage. Mechanistically, they demonstrated for the first time that endogenous glucocorticoids produced shortly after infection promote the expression of the immune checkpoint PD1 on the surface of natural killer (NK) cells. PD1 ligands are also simultaneously induced in several immune cell subsets. This glucocorticoid-PD1 pathway is tissue-specific and acts to limit the production of interferon (IFN)-γ by NK cells and to prevent lethal immunopathology following MCMV infection. Importantly, this neuroendocrine-immune regulatory axis does not impair viral clearance. The inventors thus identify a major role for the HPA axis in the promotion of host immune tolerance and resistance to an infectious disease through the regulation of the PD1 inhibitory pathway in an ILC subset. Moreover, the modulation of the glucocorticoid-PD1 pathway in order to increase or decrease the activity of ILCs would permit to treat either cancers and infectious diseases or autoimmune and inflammatory diseases.

### Methods for modulating innate lymphoid cell activity and antibody drug conjugates of the invention

A first aspect of the present invention relates to a method of modulating innate lymphoid cell activity which comprises modulating the activity of glucocorticoid receptor (not according to the invention as claimed).

As used herein, the term "subject" denotes a mammal, such as a rodent, a feline, a canine, and a primate. Preferably, a subject according to the invention is a human.

As used herein, the term "innate lymphoid cell" has its general meaning in the art and refers to a family of innate immune cells, which are part of the innate immune system, but develop from the lymphoid lineage.

In one embodiment, the innate lymphoid cell is NCR1+ innate lymphoid cell.

As used herein, the term "NCR1" refers to Natural cytotoxicity triggering receptor 1 which is a protein encoded by the NCR1 gene. NCR1 is a cytotoxicity-activating receptor.

In one embodiment, the innate lymphoid cell is natural killer.

As used herein, the term "natural killer cells" or "NK cells" has its general meaning in the art and refers to a type of cytotoxic lymphocyte of the innate immune system. Natural killer cells belong to the ILC1 family.

As used herein, the term "innate lymphoid cell activity" relates to any biological function of innate lymphoid cell including for instance cytokines secretion or cytotoxic function.

In one embodiment, the innate lymphoid cell activity is increased.

In one embodiment, the innate lymphoid cell activity is decreased.

As used herein, the term "modulating the innate lymphoid cell activity" denotes inhibiting or stimulating, partially or totally, the innate lymphoid cell activity.

As used herein, the term "increasing the innate lymphoid cell activity" denotes stimulating at least partially the innate lymphoid cell activity.

As used herein, the term "deceasing the innate lymphoid cell activity" denotes inhibiting partially or totally the innate lymphoid cell activity.

As used herein, the term "modulating the glucocorticoid receptor activity" denotes inhibiting or stimulating, partially or totally, the glucocorticoid receptor activity.

The term "glucocorticoid receptor" ("GR") also known as NR3C1 (nuclear receptor subfamily 3, group C, member 1) refers to family of intracellular receptors also referred to as the cortisol receptor, which specifically bind to cortisol and/or cortisol analogs. The term includes isoforms of GR, recombinant GR and mutated GR.

In one embodiment, the method comprises using a glucocorticoid receptor agonist or a glucocorticoid receptor antagonist.

The term "glucocorticoid receptor antagonist" or "GR antagonist" has its general meaning in the art and refers to any compound, natural or synthetic, that blocks, suppresses, or reduces (including significantly) the biological activity of GR or to any compound that inhibit GR gene expression.

The term "GR antagonist" includes but is not limited to: small organic molecule, antibody or antibody fragment, a polypeptide or an inhibitor of GR expression.

The man skilled in the art can easily identify a GR antagonist. Any technique suitable for determining the functionality of GR antagonist may be used.

For instance, a GR antagonist can be identified by carrying out the following steps: i) providing a plurality of test substances ii) determining whether the test substances are GR antagonists and iii) positively selecting the test substances that are GR antagonists.

Typically, it involves providing appropriate cells which express GR. Such cells include cells from mammals, yeast, Drosophila or E. coli. In particular, a polynucleotide encoding GR is used to transfect cells to express the receptor. The expressed receptor is then contacted with a test substance and a GR ligand, as appropriate, to observe activation of a functional response. In particular comparison steps may involve to compare the activity induced by the test substance and the activity induced by a well-known GR antagonist. In particular, substances capable of having an activity similar or even better than a well-known GR antagonist are positively selected.

Typically, it may also involve screening for test substances capable of binding to GR. Typically the test substance is labelled (e.g. with a radioactive label) and the binding is compared to a well-known GR antagonist.

Typically, the candidate compound is selected from the group consisting of small organic molecules, peptides, polypeptides or oligonucleotides.

The test substances that have been positively selected may be subjected to further selection steps in view of further assaying its properties for the treatment of inflammatory diseases, cancer, autoimmune diseases or allergy. For example, the candidate compounds that have been positively selected may be subjected to further selection steps in view of further assaying its properties on animal models.

The above assays may be performed using high throughput screening techniques for identifying test substances for developing drugs that may be useful to the treatment of these disorders. High throughput screening techniques may be carried out using multi-well plates (e.g., 96-, 389-, or 1536-well plates), in order to carry out multiple assays using an automated robotic system. Thus, large libraries of test substances may be assayed in a highly efficient manner. More particularly, stably-transfected cells growing in wells of micro-titer plates (96 well or 384 well) can be adapted to high through-put screening of libraries of compounds. Compounds in the library will be applied one at a time in an automated fashion to the wells of the microtitre dishes containing the transgenic cells described above. Once the test substances which induce the activity of GR are identified, they can be positively selected for further characterization. These assays offer several advantages. The exposure of the test substance to a whole cell allows for the evaluation of its activity in the natural context in which the test substance may act. Because this assay can readily be performed in a microtitre plate format, the assays described can be performed by an automated robotic system, allowing for testing of large numbers of test samples within a reasonably short time frame. The assays of the invention can be used as a screen to assess the activity of a previously untested compound or extract, in which case a single concentration is tested and compared to controls. These assays can also be used to assess the relative potency of a compound by testing a range of concentrations, in a range of 100 µM to 1 µM, for example, and computing the more efficient concentration.

In some embodiments, the glucocorticoid receptor antagonist is a selective glucocorticoid receptor antagonist, as set forth in Clark, 2008, which is hereby incorporated by reference. In other embodiments, the glucocorticoid receptor antagonist is a non-selective glucocorticoid receptor antagonist, such as mifepristone. In certain embodiments, the glucocorticoid receptor antagonist is steroidal. In other embodiments, the glucocorticoid receptor antagonist is nonsteroidal. A glucocorticoid receptor antagonist includes those in the following classes of chemical compounds: octahydrophenanthrenes, spirocyclic dihydropyridines, triphenylmethanes and diaryl ethers, chromenes, dibenzyl anilines, dihydroisoquinolines, pyrimidinediones, azadecalins, and aryl pyrazolo azadecalins, and which are described in more detail in Clark, 2008, which is hereby incorporated by reference. Some embodiments of steroidal antagonists from Clark, 2008 are: RU-486, RU-43044, 11-monoaryl and 11,21 bisaryl steroids (including 11β-substituted steroids), 10β-substituted steroids, 11β-aryl conjugates of mifepristone, and phosphorous-containing mifepristone analogs. Further embodiments of nonsteroidal antagonists from Clark, 2008 are: octahydrophenanthrenes, spirocyclic dihydropyridines, triphenylmethanes and diaryl ethers, chromenes, dibenzyl anilines, dihyrdroquinolines, pyrimidinediones, azadecalins, aryl pyrazolo azadecalins (including 8a-benzyl isoquinolones, N-substituted derivatives, bridgehead alcohol and ethers, bridgehead amines). Additional specific examples include, but are not limited to the following specific antagonists: beclometasone, betamethasone, budesonide, ciclesonide, flunisolide, fluticasone, mifepristone, mometasone, and triamcinolone. Other examples include those described and/or depicted in U.S. Patent Application Publication 2010/0135956, which is hereby incorporated by reference. Even further examples include ORG-34517 (Merck), RU-43044, dexamethasone mesylate (Dex-Mes), dexamethasone oxetanone (Dex-Ox), deoxycorticosterone (DOC) (Peeters et al., 2008, which is hereby incorporated by reference in its entirety and Cho et al. 2005, which is hereby incorporated by reference in its entirety). In additional embodiments the glucocorticoid receptor antagonist may be CORT 0113083 or CORT 00112716, which are described in Belanoff et al. (2011), which is hereby incorporated by reference. It is specifically contemplated that one or more of the antagonists discussed herein or in the incorporated references may be excluded in embodiments of the invention. It is also contemplated that in some embodiments, more than one glucocorticoid receptor antagonist is employed, while in other embodiments, only one is employed (though it may be administered multiple times). It is contemplated that the second one may be administered concurrently with the first one or they may be administered at different times.

The term "glucocorticoid receptor agonist" or "GR agonist" has its general meaning in the art and refers to any compound, natural or synthetic, that enhances/increases (including significantly) the biological activity of GR or to any compound that enhances/increases GR gene expression.

The term "GR agonist" includes but is not limited to: small organic molecule, antibody or antibody fragment, a polypeptide or an activator of GR expression.

The man skilled in the art can easily identify a GR agonist. Any technique suitable for determining the functionality of GR agonist may be used.

For instance, a GR agonist can be identified by carrying out the following steps: i) providing a plurality of test substances ii) determining whether the test substances are GR agonists and iii) positively selecting the test substances that are GR agonists.

In some embodiments, the GR agonist is a "selective glucocorticoid receptor agonist (SEGRA)" otherwise referred to as a "dissociated glucocorticoid receptor agonist (DIGRA)". Regarding SEGRA, reference is made to Schäcke et al., "Insight into the molecular mechanisms of glucocorticoid receptor action promotes identification of novel ligands with an improved therapeutic index," Experimental Dermatology, 2006 15:565-573, the content of which is incorporated herein by reference in its entirety.

A "SEGRA" may be described as follows. In the absence of a GR agonist, the GR resides in the cytosol in an inactive state complexed with chaperone proteins (e.g., heat shock proteins (HSPs)). Binding of GR agonists to the GR activates the GR by causing dissociation of the bound chaperones. The activated GR can then regulate gene expression via one of two pathways. (See Rhen et al., (October 2005) N. Engl. J. Med. 353(16):1711-23, which is incorporated by reference herein in its entirety). One pathway of regulation is called "transactivation" whereby the activated GR dimerizes, is translocated into the nucleus and binds to specific sequences of DNA called GR response elements and forms a complex. The GR/DNA complex recruits other proteins which transcribe downstream DNA into mRNA and eventually protein. Examples of GR responsive genes include those that encode annexin A1, angiotensin-converting enzyme, neutral endopeptidase and other anti-inflammatory proteins. The other pathway of regulation is called "transrepression" in which activated monomeric GR binds to other transcription factors such as NF-κB and AP-1 and prevents these other factors from up-regulating the expression of their target genes. These target genes encode proteins such as cyclooxygenase, NO synthase, phospholipase A2, tumor necrosis factor, transforming growth factor beta. ICAM-1, MAP kinase phosphatase MKP1, serum and glucocorticoid-inducible protein kinase SGK, FK506-binding protein FKBP51 also called immunophilin, and a number of other pro-inflammatory proteins. (See Newton et al., (October 2007) Mol. Pharmacol 72(4):799-809, which is incorporated by reference herein in its entirety). As defined herein, a "SEGRA" selectively activates the GR such that the SEGRA more strongly transrepresses than transactivates. Suitable SEGRAs for the methods disclosed herein are known in the art and may include, but are not limited to BOL-303242-X, A 276575, RU 24858, and octahydrophenanthrene-2,7-diol derivatives. (See also Mealy et al., (2009) Drugs Fut 34 (4): 341; Robinson et al. (2009) Journal of medicinal chemistry 52 (6): 1731-43; Biggadike et al., (2007) Journal of Medicinal Chemistry 50 (26): 6519; Zhang et al. (2009) Molecular Vision 15: 2606-16; Vayssiére et al., (1997) Molecular Endocrinology 11 (9): 1245-55: Lin et al., (August 2002) Molecular Pharmacology 62 (2): 297-303; Schäcke et al., (January 2004) PNAS of the United States of America 101 (1): 227-32; Newton et al., (October 2007) Mol. Pharmacol. 72 (4): 799-809; Heinemann et al., (2008) Österreichische Apothekerzeitung 62 (23); Coghlan et al., (2003) Molecular Endocrinology 17 (5): 860; Reichardt et al., (May 1998) Cell 93 (4): 531-41; Reichardt et al., (2000) Biol. Chem. 381 (9-10): 961-4; Schäcke et al., (September 2007) Molecular and Cellular Endocrinology 275 (1-2): 109-17; Schäcke et al., (200)2) Ernst Schering Research Foundation workshop (40): 357-71; Renfro et al., (1992) Dermatologic Clinics 10 (3): 505-12; and Kerscher et al. (1995) International Journal of Clinical Pharmacology and Therapeutics 33 (4): 187-9; the contents of which are incorporated herein by reference in their entireties).

In some embodiments, the GR agonist is one of agonists described in international patent application WO2009069032. In some embodiments, the GR agonist is one of agonists described in US patent application US6852719. In some embodiments, the GR agonist is one of agonists described in US patent application US20120171126.

In some embodiments, the GR agonist is fluticasone propionate.

In some embodiments, the GR agonist is GSK 9027.

In some embodiments, the GR agonist is methylprednisolone.

In some embodiments, the GR agonist is corticosterone.

In some embodiments, the GR agonist is mometasone furoate.

In some embodiment, the GR antagonist or the GR agonist is chosen from:

| Name | Chemical Name or structure | Description |
|---|---|---|
| Corticosterone | (11β)-11,21-Dihydroxypregn-4-ene-3,20-dione | Endogenous GC |
| Fluticasone propionate | (6α,11β,16α,17α)-6,9-Difluoro-11-hydroxy-16-methyl-3-oxo-17-(1-oxopropoxy)androsta-1,4-diene-17-carbothioic acid fluoromethyl ester | Selective high affinity glucocorticoid receptor agonist |
| GSK 9027 | N-[4-[1-(4-Fluorophenyl)-1H-indazol-5-yl-3-(trifluoromethyl)phenyl]benzenesulfonamide | GR agonist |
| Methylprednisolone | 11β,17β,21-Trihydroxy-6α-methyl-1,4-pregnadiene-3,20-dione | GR agonist |
| Mometasone furoate | 11β,16α)-9,21-Dichloro-11-hydroxy-16-methyl-3,20-dioxopregna-1,4-dien-17-yl 2-furoate | Synthetic corticosteroid; GC and Progesterone antagonist activity |
| Budesonide | (11β,16α)-16,17-[Butylidene*bis*(oxy)]-11,21-dihydroxypregna-1,4-diene-3,20-dione | Synthetic glucocorticoid; anti-inflammatory and chemopreventive |
| Ciclesonide | (11β,16α)-16,17-[[(*R*)-Cyclohexylmethylene]bis(oxy)]-11-hydroxy-21-(2-methyl-1-oxopropoxy)pregna-1,4-diene-3,20-dione | Glucocorticoid antiasthmatic prodrug |
| Dexamethasone | (11β,16α)-9-Fluoro-11,17,21-trihydroxy-16-methyl-pregna-1,4-diene-3,20-dione | Anti-inflammatory GC |
| Hydrocortisone | 11β,17α,21-Trihydroxypregn-4-ene-3,20-dione | Adrenal GC released in stress response |
| Prednisolone | 11,17-Dihydroxy-17-(2-hydroxyacetyl)-10,13-dimethyl-6,7,8,9,10,11,12,13,14,15,16,17-dodecahydrocyclopenta[a] phenanthren-3-one | Synthetic GC |
| Loteprednol Etabonate | 11β,17α,Dihydroxy-21-oxa-21-chloromethylpregna-1,4-diene-3,20-dione 17α-ethylcarbonate | Synthetic corticosteroid; GC antagonist activity |
| Compound A | 4-[1-Chloro-2-(methylamino)ethyl]phenyl acetate hydrochloride | Selective Glucocorticoid Recetor Agonist (SEGRA) |
| RU 24858 | 3-[(8S,9R,10S,11S,13S,14S,16R,17S)-9-fluoro-11-hydroxy-10,13,16-trimethyl-3-oxo-7,8,11,12,14,15,16,17-octahydro-6H-cyclopenta[a]phenanthren-17-yl]-3-oxopropanenitrile | Selective Glucocorticoid Recetor Agonist (SEGRA) |
| RU 24782 | (8S,9R,10S,11S,13S,14S,16R,17S)-9-fluoro-11-hydroxy-10,13,16-trimethyl-17-(2-methylsulfanylacetyl)-7,8,11,12,14,15,16,17-octahydro-6H-cyclopenta[a]phenanthren-3-one | Selective Glucocorticoid Recetor Agonist (SEGRA) |
| AL-438 | 5-Allyl-10-methoxy-2,2,4-trimethyl-2,5-dihydro-1H-6-oxa-1-aza-chrysene | Selective Glucocorticoid Recetor Agonist (SEGRA) |
| ZK 216348 | 4-(2,3-dihydro-1-benzofuran-7-yl)-2-hydroxy-4-methyl-N-(4-methyl-1-oxo-2,3-benzoxazin-6-yl)-2-(trifluoromethyl)pentanamide | Selective Glucocorticoid Recetor Agonist (SEGRA) |
| LDG-5552 | 5Z)-5-[(2-fluoro-3-methylphenyl)methylene]2,5-dihydro-10-methoxy-2,2,4-trimethyl-1*H-*(1)benzopyrano[3,4-*f*]quinolin-9-ol | Selective Glucocorticoid Recetor Agonist (SEGRA) |
| BI 653048 | 2-[(4R)-4-[(5-ethylsulfonyl-1H-pyrrolo[2,3-c]pyridin-2-yl)methyl]-5,5,5-trifluoro-4-hydroxy-2-methylpentan-2-yl]-5-fluorobenzamide | Selective Glucocorticoid Recetor Agonist (SEGRA) |
| Compound 60 | 2-[(1-Cyclopentyl-1,2,3,4-tetrahydro-1-naphthalenyl)methyl]-3,3,3-trifluoro-2-hydroxy-N-(4-methyl-1-oxo-1H-2,3-benzoxazin-6-yl)propanamide | Selective Glucocorticoid Recetor Agonist (SEGRA) |
| Compound 15 | 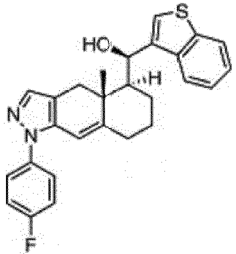 | Selective Glucocorticoid Recetor Agonist (SEGRA) |
| MK-5932 | 5-Fluoro-2-{2-[(4aS,5R)-1-(4-fluorophenyl)-5-hydroxy-4a-methyl-1,4,4a,5,6,7-hexahydrocyclopenta[f]indazol-5-yl]ethyl}benzamide | Selective Glucocorticoid Recetor Agonist (SEGRA) |
| Mapracorat | (2R)-1,1,1-trifluoro-4-(5-fluoro-2,3-dihydro-1-benzofuran-7-yl)-4-methyl-2-[[(2-methylquinolin-5-yl)amino]methyl]pentan-2-ol | Selective Glucocorticoid Recetor Agonist (SEGRA) |
| Dagrocorat | (4bS,7R,8aR)-4b-benzyl-7-hydroxy-N-(2-methylpyridin-3-yl)-7-(trifluoromethyl)-5,6,8,8a,9,10-hexahydrophenanthrene-2-carboxamide | Partial GR agonist |
| Fosdagrocorat | [(2R,4aS, 10aR)-4a-benzyl-7-[(2-methylpyridin-3-yl)carbamoyl]-2-(trifluoromethyl)-1,3,4,9,10,10a-hexahydrophenanthren-2-yl] dihydrogen phosphate | C2 dihydrogen phosphate ester of Dagrocorat |
| C108297 | (4aR)-6-(4-tert-butylphenyl)sulfonyl-1-(4-fluorophenyl)-4a-(2-methoxyethoxymethyl)-4,5,7,8-tetrahydropyrazolo[3,4-g]isoquinoline | Selective Glucocorticoid Recetor Agonist (SEGRA) |

A further aspect of the present invention relates to an antibody drug conjugate comprising an antibody that targets or binds a specific marker of innate lymphoid cell conjugated to an agonist of glucocorticoid receptor for use in a method of treating an infectious disease, wherein:
- the innate lymphoid cell is natural killer cell
- the specific marker of innate lymphoid cell is NCR1 and
- the infectious disease is caused by a virus.

The term "antibody drug conjugate" or "ADC" refers to a molecule comprising an antibody linked to a biological active compound. ADC is a class of drug designed for targeted therapy. For the purpose of the present invention, the ADC targets or binds the innate lymphoid cell and is conjugated to an agonist or to an antagonist of glucocorticoid receptor.

As used herein the term "antibody" or "immunoglobulin" have the same meaning, and will be used equally in the present invention. The term "antibody" as used herein refers to immunoglobulin molecules and immunologically active portions of immunoglobulin molecules, i.e., molecules that contain an antigen binding site that immunospecifically binds an antigen.

As used herein, the term "binds" in the context of the binding of an antibody to a predetermined antigen or epitope typically is a binding with an affinity corresponding to a KD of about 10-7 M or less, such as about 10-8 M or less, such as about 10-9 M or less, about 10-10 M or less, or about 10-11 M or even less when determined by for instance surface plasmon resonance (SPR) technology in a BIAcore 3000 instrument using a soluble form of the antigen as the ligand and the antibody as the analyte. BIACORE^{®} (GE Healthcare, Piscaataway, NJ) is one of a variety of surface plasmon resonance assay formats that are routinely used to epitope bin panels of monoclonal antibodies. Typically, an antibody binds to the predetermined antigen with an affinity corresponding to a KD that is at least ten-fold lower, such as at least 100-fold lower, for instance at least 1,000-fold lower, such as at least 10,000-fold lower, for instance at least 100,000-fold lower than its KD for binding to a non-specific antigen (e.g., BSA, casein), which is not identical or closely related to the predetermined antigen. When the KD of the antibody is very low (that is, the antibody has a high affinity), then the KD with which it binds the antigen is typically at least 10,000-fold lower than its KD for a non-specific antigen. An antibody is said to essentially not bind an antigen or epitope if such binding is either not detectable (using, for example, plasmon resonance (SPR) technology in a BIAcore 3000 instrument using a soluble form of the antigen as the ligand and the antibody as the analyte), or is 100 fold, 500 fold, 1000 fold or more than 1000 fold less than the binding detected by that antibody and an antigen or epitope having a different chemical structure or amino acid sequence.

As used herein, the term "targets" means that the ADC antibody has a specific interaction with the marker of innate lymphoid cell.

As used herein, the term "specific marker of innate lymphoid cell" refers to any molecule (for instance a protein) present specifically on ILCs. For the purpose of the present invention, the specific marker can be only present on ILCs or essentially present in ILCs.

Typically, the antibody-drug conjugate compounds comprise a linker unit between the drug unit and the antibody unit. In some embodiments, the linker is cleavable under intracellular conditions, such that cleavage of the linker releases the drug unit from the antibody in the intracellular environment. In yet other embodiments, the linker unit is not cleavable and the drug is released, for example, by antibody degradation.

In some embodiments, the linker is cleavable by a cleaving agent that is present in the intracellular environment (e.g., within a lysosome or endosome or caveolea). The linker can be, e.g., a peptidyl linker that is cleaved by an intracellular peptidase or protease enzyme, including, but not limited to, a lysosomal or endosomal protease. In some embodiments, the peptidyl linker is at least two amino acids long or at least three amino acids long. Cleaving agents can include cathepsins B and D and plasmin, all of which are known to hydrolyze dipeptide drug derivatives resulting in the release of active drug inside target cells (see, e.g., Dubowchik and Walker, 1999, Pharm. Therapeutics 83:67-123).

Most typical are peptidyl linkers that are cleavable by enzymes that are present in 191P4D12-expressing cells. Examples of such linkers are described, e.g., in U.S. Pat. No. 6,214,345, incorporated herein by reference in its entirety and for all purposes. In a specific embodiment, the peptidyl linker cleavable by an intracellular protease is a Val-Cit linker or a Phe-Lys linker (see, e.g., U.S. Pat. No. 6,214,345, which describes the synthesis of doxorubicin with the Val-Cit linker). One advantage of using intracellular proteolytic release of the therapeutic agent is that the agent is typically attenuated when conjugated and the serum stabilities of the conjugates are typically high.

In other embodiments, the cleavable linker is pH-sensitive, i.e., sensitive to hydrolysis at certain pH values.

Typically, the pH-sensitive linker hydrolyzable under acidic conditions. For example, an acid-labile linker that is hydrolyzable in the lysosome (e.g., a hydrazone, semicarbazone, thiosemicarbazone, cis-aconitic amide, orthoester, acetal, ketal, or the like) can be used. (See, e.g., U.S. Pat. Nos. 5,122,368; 5,824,805; 5,622,929; Dubowchik and Walker, 1999, Pharm. Therapeutics 83:67-123; Neville et al., 1989, Biol. Chem. 264:14653-14661.) Such linkers are relatively stable under neutral pH conditions, such as those in the blood, but are unstable at below pH 5.5 or 5.0, the approximate pH of the lysosome. In certain embodiments, the hydrolyzable linker is a thioether linker (such as, e.g., a thioether attached to the therapeutic agent via an acylhydrazone bond (see, e.g., U.S. Pat. No. 5,622,929).

In yet other embodiments, the linker is cleavable under reducing conditions (e.g., a disulfide linker). A variety of disulfide linkers are known in the art, including, for example, those that can be formed using SATA (N-succinimidyl-S-acetylthioacetate), SPDP (N-succinimidyl-3-(2-pyridyldithio)propionate), SPDB (N-succinimidyl-3-(2-pyridyldithio)butyrate) and SMPT (N-succinimidyl-oxycarbonyl-alpha-methyl-alpha-(2-pyridyl-dithio)toluene), SPDB and SMPT. (See, e.g., Thorpe et al., 1987, Cancer Res. 47:5924-5931; Wawrzynczak et al., In Immunoconjugates: Antibody Conjugates in Radioimagery and Therapy of Cancer (C. W. Vogel ed., Oxford U. Press, 1987. See also U.S. Pat. No. 4,880,935.)

In yet other specific embodiments, the linker is a malonate linker (Johnson et al., 1995, Anticancer Res. 15:1387-93), a maleimidobenzoyl linker (Lau et al., 1995, Bioorg-Med-Chem. 3(10):1299-1304), or a 3' -N-amide analog (Lau et al., 1995, Bioorg-Med-Chem. 3(10):1305-12).

In yet other embodiments, the linker unit is not cleavable and the drug is released by antibody degradation.

Typically, the linker is not substantially sensitive to the extracellular environment. As used herein, "not substantially sensitive to the extracellular environment," in the context of a linker, means that no more than about 20 %, typically no more than about 15 %, more typically no more than about 10 %, and even more typically no more than about 5 %, no more than about 3 %, or no more than about 1 % of the linkers, in a sample of antibody-drug conjugate compound, are cleaved when the antibody-drug conjugate compound is present in an extracellular environment (e.g., in plasma). Whether a linker is not substantially sensitive to the extracellular environment can be determined, for example, by incubating with plasma the antibody-drug conjugate compound for a predetermined time period (e.g., 2, 4, 8, 16, or 24 hours) and then quantitating the amount of free drug present in the plasma.

Techniques for conjugating molecules to antibodies, are well-known in the art (See, e.g., Arnon et al., "Monoclonal Antibodies For Immunotargeting Of Drugs In Cancer Therapy," in Monoclonal Antibodies And Cancer Therapy (Reisfeld et al. eds., Alan R. Liss, Inc., 1985); Hellstrom et al., "Antibodies For Drug Delivery," in Controlled Drug Delivery (Robinson et al. eds., Marcel Deiker, Inc., 2nd ed. 1987); Thorpe, "Antibody Carriers Of Cytotoxic Agents In Cancer Therapy: A Review," in Monoclonal Antibodies '84: Biological And Clinical Applications (Pinchera et al. eds., 1985); "Analysis, Results, and Future Prospective of the Therapeutic Use of Radiolabeled Antibody In Cancer Therapy," in Monoclonal Antibodies For Cancer Detection And Therapy (Baldwin et al. eds., Academic Press, 1985); and Thorpe et al., 1982, Immunol. Rev. 62:119-58. See also, e.g., PCT publication WO 89/12624.) Typically, the molecule is covalently attached to lysines or cysteines on the antibody, through N-hydroxysuccinimide ester or maleimide functionality respectively. Methods of conjugation using engineered cysteines or incorporation of unnatural amino acids have been reported to improve the homogeneity of the conjugate (Axup, J.Y., Bajjuri, K.M., Ritland, M., Hutchins, B.M., Kim, C.H., Kazane, S.A., Halder, R., Forsyth, J.S., Santidrian, A.F., Stafin, K., et al.

(2012). Synthesis of site-specific antibody-drug conjugates using unnatural amino acids. Proc. Natl. Acad. Sci. USA 109, 16101-16106.; Junutula, J.R., Flagella, K.M., Graham, R.A., Parsons, K.L., Ha, E., Raab, H., Bhakta, S., Nguyen, T., Dugger, D.L., Li, G., et al. (2010). Engineered thio-trastuzumab-DM1 conjugate with an improved therapeutic index to target humanepidermal growth factor receptor 2-positive breast cancer. Clin. Cancer Res.16, 4769-4778.). Junutula et al. (2008) developed cysteine-based site-specific conjugation called "THIOMABs" (TDCs) that are claimed to display an improved therapeutic index as compared to conventional conjugation methods. Conjugation to unnatural amino acids that have been incorporated into the antibody is also being explored for ADCs; however, the generality of this approach is yet to be established (Axup et al., 2012). In particular the one skilled in the art can also envisage Fc-containing polypeptide engineered with an acyl donor glutamine-containing tag (e.g., Gin-containing peptide tags or Q- tags) or an endogenous glutamine that are made reactive by polypeptide engineering (e.g., via amino acid deletion, insertion, substitution, or mutation on the polypeptide). Then a transglutaminase, can covalently crosslink with an amine donor agent (e.g., a small molecule comprising or attached to a reactive amine) to form a stable and homogenous population of an engineered Fc-containing polypeptide conjugate with the amine donor agent being site- specifically conjugated to the Fc-containing polypeptide through the acyl donor glutamine- containing tag or the accessible/exposed/reactive endogenous glutamine (WO 2012059882).

### Therapeutic uses of the methods of the invention

Disclosed but not according to the invention as claimed is a method of treating cancer in a subject in need thereof comprising performing the method of the invention consisting of modulating innate lymphoid cell activity which comprises modulating the activity of glucocorticoid receptor. In particular, the innate lymphoid cell activity is increased using the method of he invention.

The terms "cancer" has its general meaning in the art and refers to a group of diseases involving abnormal cell growth with the potential to invade or spread to other parts of the body. The term "cancer" further encompasses both primary and metastatic cancers. Examples of cancers that may treated by methods and compositions of the invention include, but are not limited to, cancer cells from the bladder, blood, bone, bone marrow, brain, breast, colon, esophagus, gastrointestinal, gum, head, kidney, liver, lung, nasopharynx, neck, ovary, prostate, skin, stomach, testis, tongue, or uterus. In addition, the cancer may specifically be of the following histological type, though it is not limited to these: neoplasm, malignant; carcinoma; carcinoma, undifferentiated; giant and spindle cell carcinoma; small cell carcinoma; papillary carcinoma; squamous cell carcinoma; lymphoepithelial carcinoma; basal cell carcinoma; pilomatrix carcinoma; transitional cell carcinoma; papillary transitional cell carcinoma; adenocarcinoma; gastrinoma, malignant; cholangiocarcinoma; hepatocellular carcinoma; combined hepatocellular carcinoma and cholangiocarcinoma; trabecular adenocarcinoma; adenoid cystic carcinoma; adenocarcinoma in adenomatous polyp; adenocarcinoma, familial polyposis coli; solid carcinoma; carcinoid tumor, malignant; branchiolo-alveolar adenocarcinoma; papillary adenocarcinoma; chromophobe carcinoma; acidophil carcinoma; oxyphilic adenocarcinoma; basophil carcinoma; clear cell adenocarcinoma; granular cell carcinoma; follicular adenocarcinoma; papillary and follicular adenocarcinoma; non encapsulating sclerosing carcinoma; adrenal cortical carcinoma; endometroid carcinoma; skin appendage carcinoma; apocrine adenocarcinoma; sebaceous adenocarcinoma; ceruminous; adenocarcinoma; mucoepidermoid carcinoma; cystadenocarcinoma; papillary cystadenocarcinoma; papillary serous cystadenocarcinoma; mucinous cystadenocarcinoma; mucinous adenocarcinoma; signet ring cell carcinoma; infiltrating duct carcinoma; medullary carcinoma; lobular carcinoma; inflammatory carcinoma; paget's disease, mammary; acinar cell carcinoma; adenosquamous carcinoma; adenocarcinoma w/squamous metaplasia; thymoma, malignant; ovarian stromal tumor, malignant; thecoma, malignant; granulosa cell tumor, malignant; and roblastoma, malignant; Sertoli cell carcinoma; leydig cell tumor, malignant; lipid cell tumor, malignant; paraganglioma, malignant; extra-mammary paraganglioma, malignant; pheochromocytoma; glomangiosarcoma; malignant melanoma; amelanotic melanoma; superficial spreading melanoma; malign melanoma in giant pigmented nevus; epithelioid cell melanoma; blue nevus, malignant; sarcoma; fibrosarcoma; fibrous histiocytoma, malignant; myxosarcoma; liposarcoma; leiomyosarcoma; rhabdomyosarcoma; embryonal rhabdomyosarcoma; alveolar rhabdomyosarcoma; stromal sarcoma; mixed tumor, malignant; mullerian mixed tumor; nephroblastoma; hepatoblastoma; carcinosarcoma; mesenchymoma, malignant; brennertumor, malignant; phyllodestumor, malignant; synovial sarcoma; mesothelioma, malignant; dysgerminoma; embryonal carcinoma; teratoma, malignant; strumaovarii, malignant; choriocarcinoma; mesonephroma, malignant; hemangiosarcoma; hemangioendothelioma, malignant; kaposi's sarcoma; hemangiopericytoma, malignant; lymphangiosarcoma; osteosarcoma; juxtacortical osteosarcoma; chondrosarcoma; chondroblastoma, malignant; mesenchymal chondrosarcoma; giant cell tumor of bone; ewing's sarcoma; odontogenic tumor, malignant; ameloblasticodontosarcoma; ameloblastoma, malignant; ameloblasticfibrosarcoma; pinealoma, malignant; chordoma; glioma, malignant; ependymoma; astrocytoma; protoplasmic astrocytoma; fibrillary astrocytoma; astroblastoma; glioblastoma; oligodendroglioma; oligodendroblastoma; primitive neuroectodermal; cerebellar sarcoma; ganglioneuroblastoma; neuroblastoma; retinoblastoma; olfactory neurogenic tumor; meningioma, malignant; neurofibrosarcoma; neurilemmoma, malignant; granular cell tumor, malignant; malignant lymphoma; Hodgkin's disease; Hodgkin's lymphoma; paragranuloma; malignant lymphoma, small lymphocytic; malignant lymphoma, large cell, diffuse; malignant lymphoma, follicular; mycosis fungoides; other specified non-Hodgkin's lymphomas; malignant histiocytosis; multiple myeloma; mast cell sarcoma; immunoproliferative small intestinal disease; leukemia; lymphoid leukemia; plasma cell leukemia; erythroleukemia; lymphosarcoma cell leukemia; myeloid leukemia; basophilic leukemia; eosinophilic leukemia; monocyticleukemia; mast cell leukemia; megakaryoblasticleukemia; myeloid sarcoma; and hairy cell leukemia.

In some embodiments, the subject suffers from a cancer selected from the group consisting of bile duct cancer, bladder cancer, bone cancer, brain and central nervous system cancer, breast cancer, Castleman disease cervical cancer, colorectal cancer, endometrial cancer, esophagus cancer, gallbladder cancer, gastrointestinal carcinoid tumors, Hodgkin's disease, non-Hodgkin's lymphoma, Kaposi's sarcoma, kidney cancer, laryngeal and hypopharyngeal cancer, liver cancer, lung cancer, mesothelioma, plasmacytoma, nasal cavity and paranasal sinus cancer, nasopharyngeal cancer, neuroblastoma, oral cavity and oropharyngeal cancer, ovarian cancer, pancreatic cancer, penile cancer, pituitary cancer, prostate cancer, retinoblastoma, rhabdomyosarcoma, salivary gland cancer, skin cancer, stomach cancer, testicular cancer, thymus cancer, thyroid cancer, vaginal cancer, vulvar cancer, and uterine cancer.

Another aspect of the present invention relates to the ADC as claimed for use in a method of treating infectious diseases in a subject in need thereof comprising performing the method of the invention consisting of modulating innate lymphoid cell activity which comprises modulating the activity of glucocorticoid receptor. In particular, the innate lymphoid cell activity is increased using the method of the invention.

The term "infectious disease" refers to a disease caused by an infectious agent. The term "infectious agent" is intended to mean pathogenic microorganisms, such as bacteria, viruses, fungi and intra- or extra-cellular parasites

The infectious diseases according to the invention are caused by a virus

Another aspect of the present invention relates to a method of treating inflammatory diseases in a subject in need thereof comprising performing the method of the invention consisting of modulating innate lymphoid cell activity which comprises modulating the activity of glucocorticoid receptor (not according to the invention as claimed). In particular, the innate lymphoid cell activity is decreased using the method of the invention.

As used herein the term "inflammatory disease" refers to any disease associated with inflammation. Inflammatory diseases includes, but are not limited to, rheumatoid arthritis, osteoarthritis, juvenile idiopathic arthritis, psoriasis, allergic airway disease (e.g. asthma, rhinitis), inflammatory bowel diseases (e.g. Crohn's disease, colitis), endotoxin-driven disease states (e.g. complications after bypass surgery or chronic endotoxin states contributing to e.g. chronic cardiac failure), and related diseases involving cartilage, such as that of the joints.

Another aspect of the present invention relates to a method of treating autoimmune diseases in a subject in need thereof comprising performing the method of the invention consisting of modulating innate lymphoid cell activity which comprises modulating the activity of glucocorticoid receptor (not according to the invention as claimed). In particular, the innate lymphoid cell activity is decreased using the method of the invention.

As used herein, the term "autoimmune disease" refers to the presence of an autoimmune response (an immune response directed against an auto- or self-antigen) in a subject. Autoimmune diseases include diseases caused by a breakdown of self-tolerance such that the adaptive immune system, in concert with cells of the innate immune system, responds to self-antigens and mediates cell and tissue damage. In some embodiments, autoimmune diseases are characterized as being a result of, at least in part, a humoral and/or cellular immune response. Examples of autoimmune disease include, without limitation, acute disseminated encephalomyelitis (ADEM), acute necrotizing hemorrhagic leukoencephalitis, Addison's disease, agammaglobulinemia, alopecia areata, amyloidosis, ankylosing spondylitis, anti-GBM/Anti-TBM nephritis, antiphospholipid syndrome (APS), autoimmune angioedema, autoimmune aplastic anemia, autoimmune dysautonomia, autoimmune hepatitis, autoimmune hyperlipidemia, autoimmune immunodeficiency, autoimmune inner ear disease (AIED), autoimmune myocarditis, autoimmune pancreatitis, autoimmune retinopathy, autoimmune thrombocytopenic purpura (ATP), autoimmune thyroid disease, autoimmune urticaria, axonal and neuronal neuropathies, Behcet's disease, bullous pemphigoid, autoimmune cardiomyopathy, Castleman disease, celiac disease, Chagas disease, chronic fatigue syndrome, chronic inflammatory demyelinating polyneuropathy (CIDP), chronic recurrent multifocal ostomyelitis (CRMO), Churg-Strauss syndrome, cicatricial pemphigoid/benign mucosal pemphigoid, Crohn's disease, Cogans syndrome, cold agglutinin disease, congenital heart block, coxsackie myocarditis, CREST disease, essential mixed cryoglobulinemia, demyelinating neuropathies, dermatitis herpetiformis, dermatomyositis, Devic's disease (neuromyelitis optica), discoid lupus, Dressler's syndrome, endometriosis, eosinophilic fasciitis, erythema nodosum, experimental allergic encephalomyelitis, Evans syndrome, fibromyalgia, fibrosing alveolitis, giant cell arteritis (temporal arteritis), glomerulonephritis, Goodpasture's syndrome, granulomatosis with polyangiitis (GPA), Graves' disease, Guillain-Barre syndrome, Hashimoto's encephalitis, Hashimoto's thyroiditis, hemolytic anemia, Henoch-Schonlein purpura, herpes gestationis, hypogammaglobulinemia, hypergammaglobulinemia, idiopathic thrombocytopenic purpura (ITP), IgA nephropathy, IgG4-related sclerosing disease, immunoregulatory lipoproteins, inclusion body myositis, inflammatory bowel disease, insulin-dependent diabetes (type 1), interstitial cystitis, juvenile arthritis, Kawasaki syndrome, Lambert-Eaton syndrome, leukocytoclastic vasculitis, lichen planus, lichen sclerosus, ligneous conjunctivitis, linear IgA disease (LAD), lupus (SLE), Lyme disease, Meniere's disease, microscopic polyangiitis, mixed connective tissue disease (MCTD), monoclonal gammopathy of undetermined significance (MGUS), Mooren's ulcer, Mucha-Habermann disease, multiple sclerosis, myasthenia gravis, myositis, narcolepsy, neuromyelitis optica (Devic's), autoimmune neutropenia, ocular cicatricial pemphigoid, optic neuritis, palindromic rheumatism, PANDAS (Pediatric Autoimmune Neuropsychiatric Disorders Associated with Streptococcus), paraneoplastic cerebellar degeneration, paroxysmal nocturnal hemoglobinuria (PNH), Parry Romberg syndrome, Parsonnage-Turner syndrome, pars planitis (peripheral uveitis), pemphigus, peripheral neuropathy, perivenous encephalomyelitis, pernicious anemia, POEMS syndrome, polyarteritis nodosa, type **I, II, & III** autoimmune polyglandular syndromes, polymyalgia rheumatica, polymyositis, postmyocardial infarction syndrome, postpericardiotomy syndrome, progesterone dermatitis, primary biliary cirrhosis, primary sclerosing cholangitis, psoriasis, psoriatic arthritis, idiopathic pulmonary fibrosis, pyoderma gangrenosum, pure red cell aplasia, Raynaud's phenomenon, reflex sympathetic dystrophy, Reiter's syndrome, relapsing polychondritis, restless legs syndrome, retroperitoneal fibrosis, rheumatic fever, rheumatoid arthritis, sarcoidosis, Schmidt syndrome, scleritis, scleroderma, Sjogren's syndrome, sperm & testicular autoimmunity, stiff person syndrome, subacute bacterial endocarditis (SBE), Susac's syndrome, sympathetic ophthalmia, Takayasu's arteritis, temporal arteritis/Giant cell arteritis, thrombocytopenic purpura (TTP), Tolosa-Hunt syndrome, transverse myelitis, ulcerative colitis, undifferentiated connective tissue disease (UCTD), uveitis, vasculitis, vesiculobullous dermatosis, vitiligo, Waldenstrom's macroglobulinemia (WM), and Wegener's granulomatosis [Granulomatosis with Polyangiitis (GPA)]. In some embodiments, the autoimmune disease is selected from the group consisting of rheumatoid arthritis, type 1 diabetes, systemic lupus erythematosus (lupus or SLE), myasthenia gravis, multiple sclerosis, scleroderma, Addison's Disease, bullous pemphigoid, pemphigus vulgaris, Guillain-Barré syndrome, Sjogren syndrome, dermatomyositis, thrombotic thrombocytopenic purpura, hypergammaglobulinemia, monoclonal gammopathy of undetermined significance (MGUS), Waldenstrom's macroglobulinemia (WM), chronic inflammatory demyelinating polyradiculoneuropathy (CIDP), Hashimoto's Encephalopathy (HE), Hashimoto's Thyroiditis, Graves' Disease, Wegener's Granulomatosis [Granulomatosis with Polyangiitis (GPA)].

Another aspect of the present invention relates to a method of treating allergy in a subject in need thereof comprising performing the method of the invention consisting of modulating innate lymphoid cell activity which comprises modulating the activity of glucocorticoid receptor (not according to the invention as claimed). In particular, the innate lymphoid cell activity is decreased using the method of the invention.

As used herein, the term "allergy" generally refers to an inappropriate immune response characterized by inflammation and includes, without limitation, food allergies, respiratory allergies and other allergies causing or with the potential to cause a systemic response such as, by way of example, Quincke's oedema and anaphylaxis. The term encompasses allergy, allergic disease, hypersensitive associated disease or respiratory disease associated with airway inflammation, such as asthma or allergic rhinitis. In some embodiments, the method of the present invention is effective in preventing, treating or alleviating one or more symptoms related to anaphylaxis, drug hypersensitivity, skin allergy, eczema, allergic rhinitis, urticaria, atopic dermatitis, dry eye disease, allergic contact allergy, food hypersensitivity, allergic conjunctivitis, insect venom allergy, bronchial asthma, allergic asthma, intrinsic asthma, occupational asthma, atopic asthma, acute respiratory distress syndrome (ARDS) and chronic obstructive pulmonary disease (COPD). Hypersensitivity associated diseases or disorders that may be treated by the method of the present invention include, but are not limited to, anaphylaxis, drug reactions, skin allergy, eczema, allergic rhinitis, urticaria, atopic dermatitis, dry eye disease [or otherwise referred to as Keratoconjunctivitis sicca (KCS), also called keratitis sicca, xerophthalmia], allergic contact allergy, food allergy, allergic conjunctivitis, insect venom allergy and respiratory diseases associated with airway inflammation, for example, IgE mediated asthma and non-IgE mediated asthma. The respiratory diseases associated with airway inflammation may include, but are not limited to, rhinitis, allergic rhinitis, bronchial asthma, allergic (extrinsic) asthma, non-allergic (intrinsic) asthma, occupational asthma, atopic asthma, exercise induced asthma, cough-induced asthma, acute respiratory distress syndrome (ARDS) and chronic obstructive pulmonary disease (COPD).

Another aspect of the present invention relates to a method of treating immune reactions against molecules that are exogenously administered in a subject in need thereof comprising performing the method of the invention consisting of modulating innate lymphoid cell activity which comprises modulating the activity of glucocorticoid receptor. In particular, the innate lymphoid cell activity is decreased using the method of the invention.

Non-limiting examples of this kind include immune reactions against replacement therapeutics in the context of genetic deficiencies, which include, but are not limited to, haemophilia A, haemophilia B, congenital deficiency of other clotting factors such as factor II, prothrombin and fibrinogen, primary immunodeficiencies (e.g. severe combined immunodeficiency, X-linked agammaglobulinemia, IgA deficiency), primary hormone deficiencies such as growth hormone deficiency and leptin deficiency, congenital enzymopathies and metabolic disorders such as disorders of carbohydrate metabolism (e.g. sucrose-isomaltase deficiency, glycogen storage diseases), disorders of amino acid metabolism (e.g. phenylketonuria, maple syrup urine disease, glutaric acidemia type 1), urea cycle disorders (e.g. carbamoyl phosphate synthetase I deficiency), disorders of organic acid metabolism (e.g. alcaptonuria, 2-hydroxyglutaric acidurias), disorders of fatty acid oxidation and mitochondrial metabolism (e.g. medium-chain acyl-coenzyme A dehydrogenase deficiency), disorders of porphyrin metabolism (e.g. porphyrias), disorders of purine or pyrimidine metabolism (e.g. Lesch-Nyhan syndrome), disorders of steroid metabolism (e.g. lipoid congenital adrenal hyperplasia, congenital adrenal hyperplasia), disorders of mitochondrial function (e.g. Kearns-Sayre syndrome), disorders of peroxisomal function (e.g. Zellweger syndrome), lysosomal storage disorders (e.g. Gaucher's disease, Niemann Pick disease).

Another aspect of the present invention relates to a method of treating immune reactions against a grafted tissue or grafted cells in a subject in need thereof comprising performing the method of the invention consisting of modulating innate lymphoid cell activity which comprises modulating the activity of glucocorticoid receptor (not according to the invention as claimed). In particular, the innate lymphoid cell activity is decreased using the method of the invention.

As used herein, the term "grafted" refers to organs and/or tissues and/or cells which can be obtained from a first organism (or donor) and transplanted into a second organism (or recipient.

Typically the subject may have been transplanted with a graft selected from the group consisting of heart, kidney, lung, liver, pancreas, pancreatic islets, brain tissue, stomach, large intestine, small intestine, cornea, skin, trachea, bone, bone marrow, muscle, or bladder. The method of the present invention is also particularly suitable for treating an immune response associated with rejection of a donor tissue, cell, graft, or organ transplant by a recipient subject. Graft-related diseases or disorders include graft versus host disease (GVHD), such as associated with bone marrow transplantation, and immune disorders resulting from or associated with rejection of organ, tissue, or cell graft transplantation (e.g., tissue or cell allografts or xenografts), including e.g., grafts of skin, muscle, neurons, islets, organs, parenchymal cells of the liver, etc. Thus the method of the invention is useful for preventing Host-Versus-Graft-Disease (HVGD) and Graft-Versus-Host-Disease (GVHD).

As used herein, "treatment" or "treating" is an approach for obtaining beneficial or desired results including clinical results. For purposes of this invention, beneficial or desired clinical results include, but are not limited to, one or more of the following: alleviating one or more symptoms resulting from the disease, diminishing the extent of the disease, stabilizing the disease (e.g., preventing or delaying the worsening of the disease), preventing or delaying the spread of the disease, preventing or delaying the recurrence of the disease, delaying or slowing the progression of the disease, ameliorating the disease state, providing a remission (partial or total) of the disease, decreasing the dose of one or more other medications required to treat the disease, delaying the progression of the disease, increasing the quality of life, and/or prolonging survival. The term "treatment" encompasses the prophylactic treatment. As used herein, the term "prevent" refers to the reduction in the risk of acquiring or developing a given condition.

### Pharmaceutical compositions

An aspect of the present invention relates to a pharmaceutical composition comprising the antibody drug conjugate of the invention.

Typically, the antibody drug conjugate of the present invention is administered to the subject in the form of a pharmaceutical composition which comprises a pharmaceutically acceptable carrier. Pharmaceutically acceptable carriers that may be used in these compositions include, but are not limited to, ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene- block polymers, polyethylene glycol and wool fat. For use in administration to a patient, the composition will be formulated for administration to the patient. The compositions of the present invention may be administered orally, parenterally, by inhalation spray, topically, rectally, nasally, buccally, vaginally or via an implanted reservoir. The used herein includes subcutaneous, intravenous, intramuscular, intra-articular, intra-synovial, intrasternal, intrathecal, intrahepatic, intralesional and intracranial injection or infusion techniques. Sterile injectable forms of the compositions of this invention may be aqueous or an oleaginous suspension. These suspensions may be formulated according to techniques known in the art using suitable dispersing or wetting agents and suspending agents. The sterile injectable preparation may also be a sterile injectable solution or suspension in a non-toxic parenterally acceptable diluent or solvent, for example as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium. For this purpose, any bland fixed oil may be employed including synthetic mono-or diglycerides. Fatty acids, such as oleic acid and its glyceride derivatives are useful in the preparation of injectables, as are natural pharmaceutically-acceptable oils, such as olive oil or castor oil, especially in their polyoxyethylated versions. These oil solutions or suspensions may also contain a long-chain alcohol diluent or dispersant, such as carboxymethyl cellulose or similar dispersing agents that are commonly used in the formulation of pharmaceutically acceptable dosage forms including emulsions and suspensions. Other commonly used surfactants, such as Tweens, Spans and other emulsifying agents or bioavailability enhancers which are commonly used in the manufacture of pharmaceutically acceptable solid, liquid, or other dosage forms may also be used for the purposes of formulation. The compositions of this invention may be orally administered in any orally acceptable dosage form including, but not limited to, capsules, tablets, aqueous suspensions or solutions. In the case of tablets for oral use, carriers commonly used include lactose and corn starch. Lubricating agents, such as magnesium stearate, are also typically added. For oral administration in a capsule form, useful diluents include, e.g., lactose. If desired, certain sweetening, flavoring or coloring agents may also be added. Alternatively, the compositions of this invention may be administered in the form of suppositories for rectal administration. These can be prepared by mixing the agent with a suitable non-irritating excipient that is solid at room temperature but liquid at rectal temperature and therefore will melt in the rectum to release the drug. Such materials include cocoa butter, beeswax and polyethylene glycols. The compositions of this invention may also be administered by nasal aerosol or inhalation. Such compositions are prepared according to techniques well-known in the art of pharmaceutical formulation and may be prepared as solutions in saline, employing benzyl alcohol or other suitable preservatives, absorption promoters to enhance bioavailability, fluorocarbons, and/or other conventional solubilizing or dispersing agents. For example, an antibody drug conjugate present in a pharmaceutical composition of this invention can be supplied at a concentration of 10 mg/mL in either 100 mg (10 mL) or 500 mg (50 mL) single-use vials. The product is formulated for IV administration in 9.0 mg/mL sodium chloride, 7.35 mg/mL sodium citrate dihydrate, 0.7 mg/mL polysorbate 80, and Sterile Water for Injection. The pH is adjusted to 6.5. An exemplary suitable dosage range for an antibody drug conjugate in a pharmaceutical composition of this invention may between about 1 mg/m2 and 500 mg/m2. However, it will be appreciated that these schedules are exemplary and that an optimal schedule and regimen can be adapted taking into account the affinity and tolerability of the particular antibody drug conjugate in the pharmaceutical composition that must be determined in clinical trials. A pharmaceutical composition of the invention for injection (e.g., intramuscular, i.v.) could be prepared to contain sterile buffered water (e.g. 1 ml for intramuscular), and between about 1 ng to about 100 mg, e.g. about 50 ng to about 30 mg or more preferably, about 5 mg to about 25 mg.

The invention will be further illustrated by the following figures and examples. However, these examples and figures should not be interpreted in any way as limiting the scope of the present invention.

### FIGURES:

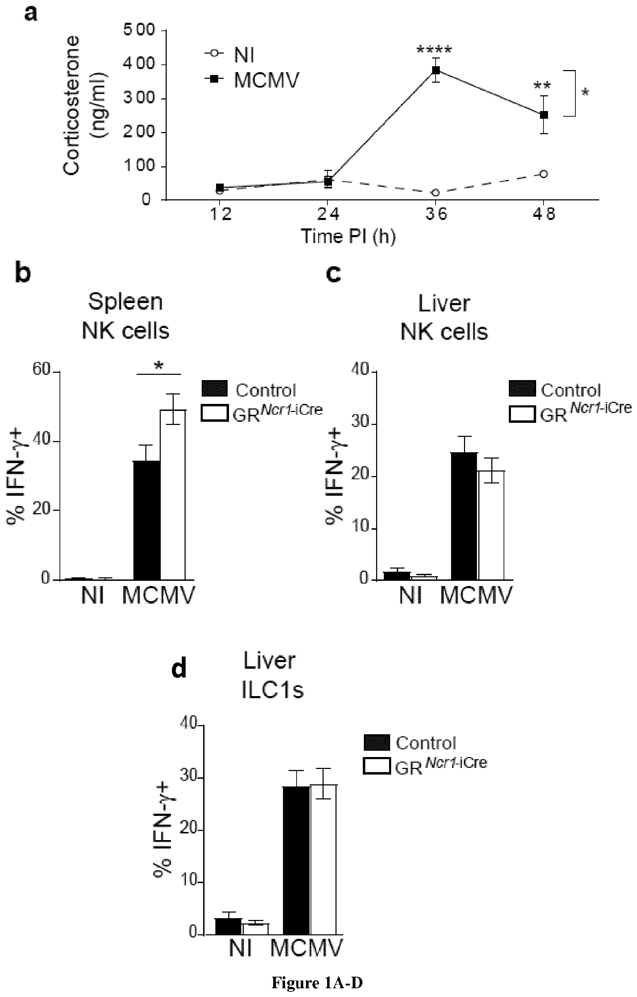

**Figure 1****: Organ-specific glucocorticoid regulation of group 1 ILC IFN-γ production upon MCMV infection. a,** Corticosterone concentration in the serum of control mice receiving injections of MCMV or DMEM (NI, non-infected) (means ± SEM, **P*<0.05, ***P<0.01,* *****P*<0.0001, one-way ANOVA) (*n*=5, pool of 2 experiments). **b-d,** Intracellular IFN-γ production was assessed directly *ex vivo* without *in vitro* restimulation. Frequency of IFN-γ-producing spleen NK cells (**b**, *n*=6-17 pool of 5 experiments), liver NK cells (**c**, n=6-16 pool of 5 experiments) and liver ILC1s (**d**, *n*=6-16 pool of 5 experiments) 44 h post-infection (PI) are shown (means ± SEM and representative FACS plots, **P*<0.05, Student's *t*-test).

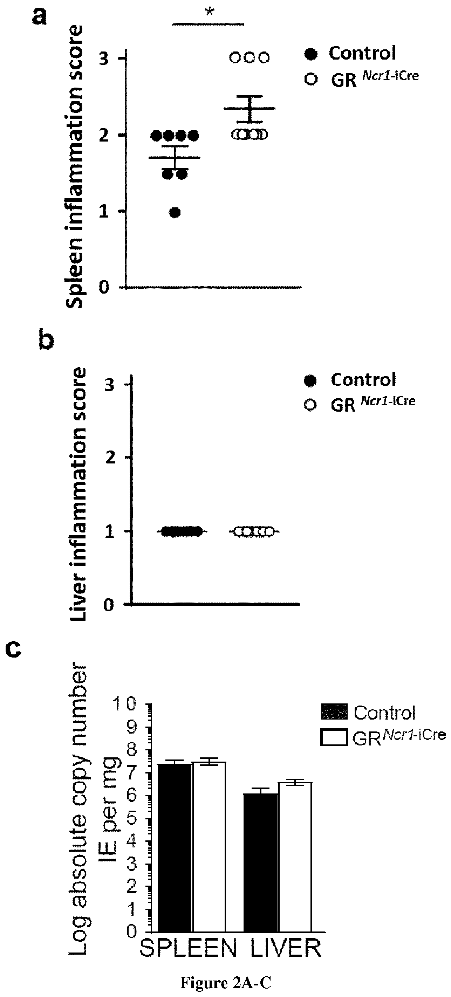

**Figure 2****: Spleens of GR**^{*Ncr1-i*Cre} **mice display more marked inflammation but unchanged viral titer. a** and **b,** quantification of spleen **(a)** and liver **(b)** inflammation 44 h PI: 1=mild, 2=moderate and 3=marked (*n*=7-9, pool of 2 experiments. Each symbol represents an individual mouse) (means ± SEM, **P*<0.05, Mann-Whitney test). **c,** Viral titer at 44 h PI (*n* =6-8, pool of 2 experiments) (means ± SEM).

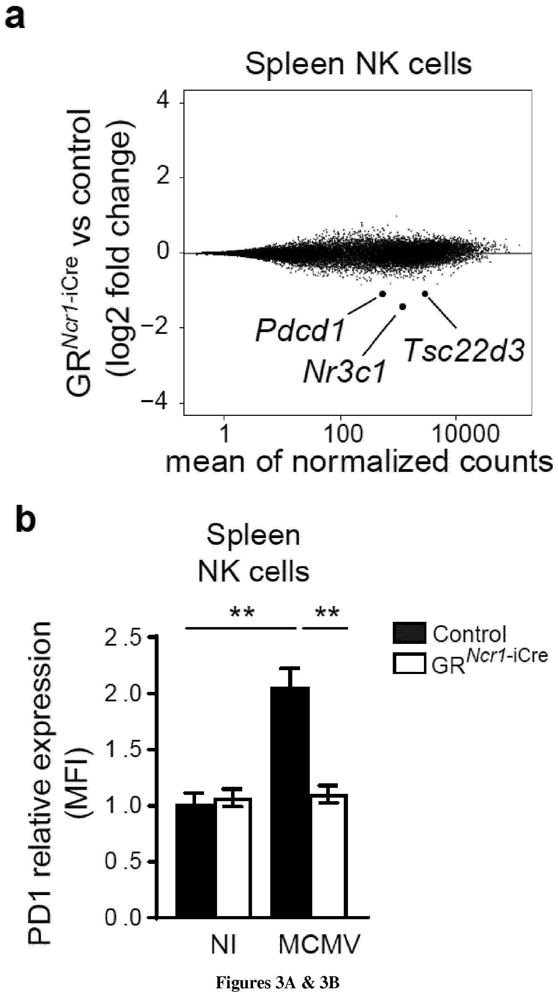

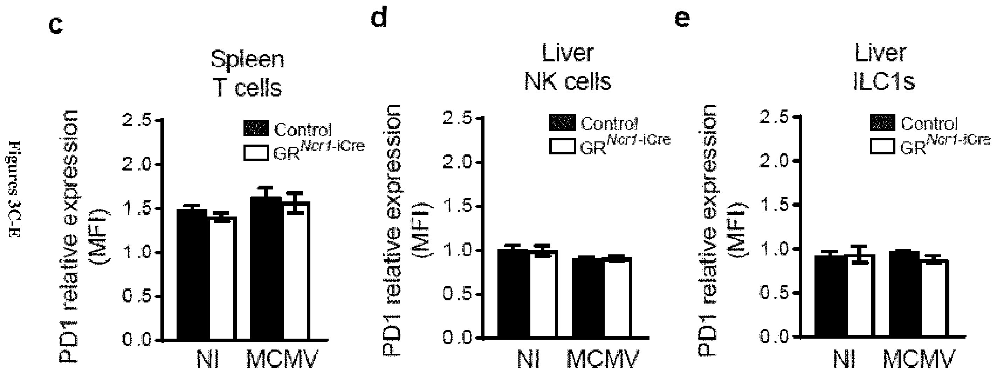

**Figure 3****: MCMV infection induces the glucocorticoid-dependent expression of PD1 in spleen NK cells. a.** RNA Seq analysis. MA plot of genes differentially expressed between spleen NK cells sorted from GR^{*Ncr1-i*Cre} and control mice 44 h PI. Genes with significantly lower levels of expression in GR-deficient NK cells are highlighted (absolute log₂ fold-change>1 and *P*<0.05) (*n*=3 samples GR^{*Ncr1*-iCre} versus 3 control samples). **b-e** FACS analysis of PD1 cell surface expression in spleen NK cells (**b**, *n*=6-12, pool of 3 experiments), spleen T cells (**c**, *n*=2-6, pool of 2 experiments), liver NK cells (**d**, *n*=3-8, pool of 2 experiments) and liver ILC1s (**e**, *n*=2-6, pool of 2 experiments) shown as MFI (mean fluorescence intensity) ratio relative to isotype control (means ± SEM, ***P*<0.01, one-way ANOVA).

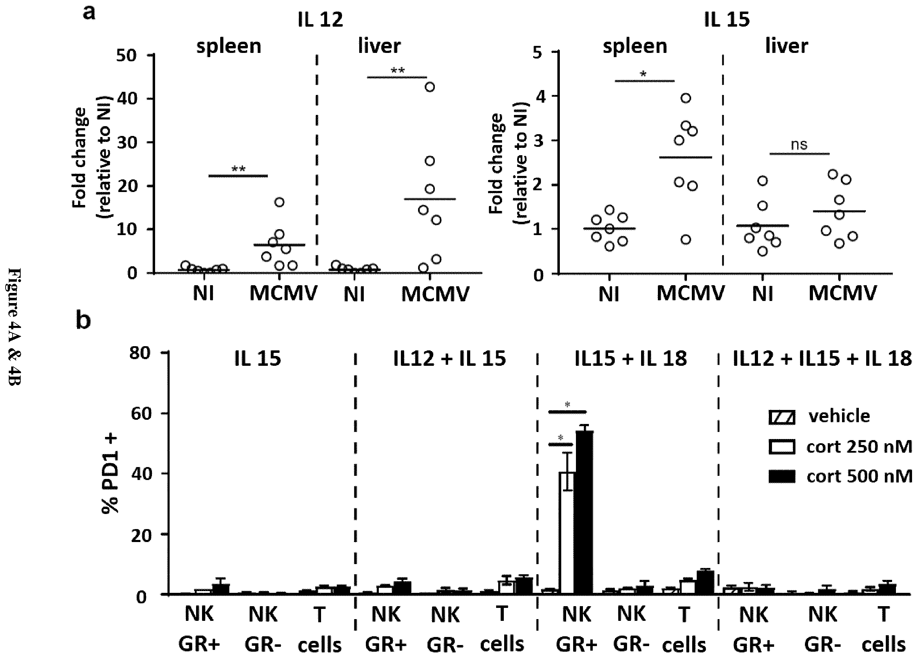

**Figure 4****: Specific cytokine combination and corticosterone cooperate to induce PD1 expression on NK cells. a,** RT-PCR of *il-12* and *il-15* from RNA extracted from spleen and liver homogenates 44h PI (*n*=7, pool of 2 experiments. Each symbol represents an individual mouse) (**P*<0.05, ***P<0.01,* ns=not significant, Mann-Whitney test). **b,** splenocytes form control and **GR**^{*Ncr1-i*Cre} mice were stimulated in vitro for 4h at 37°C with the cytokines indicated in the presence of corticosterone or vehicle alone. Means ± SEM (*n*=3 experiments, **P*<0.05, one-way ANOVA).

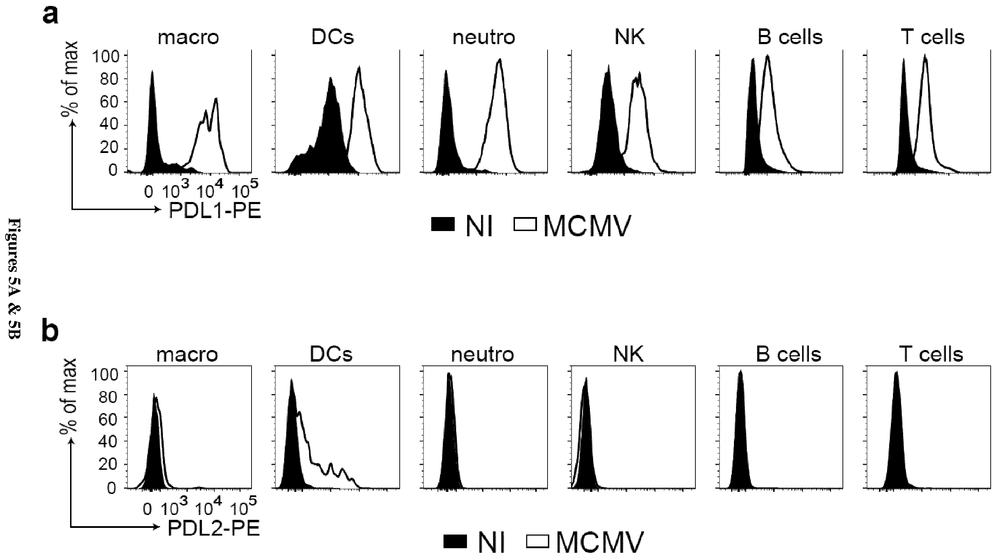

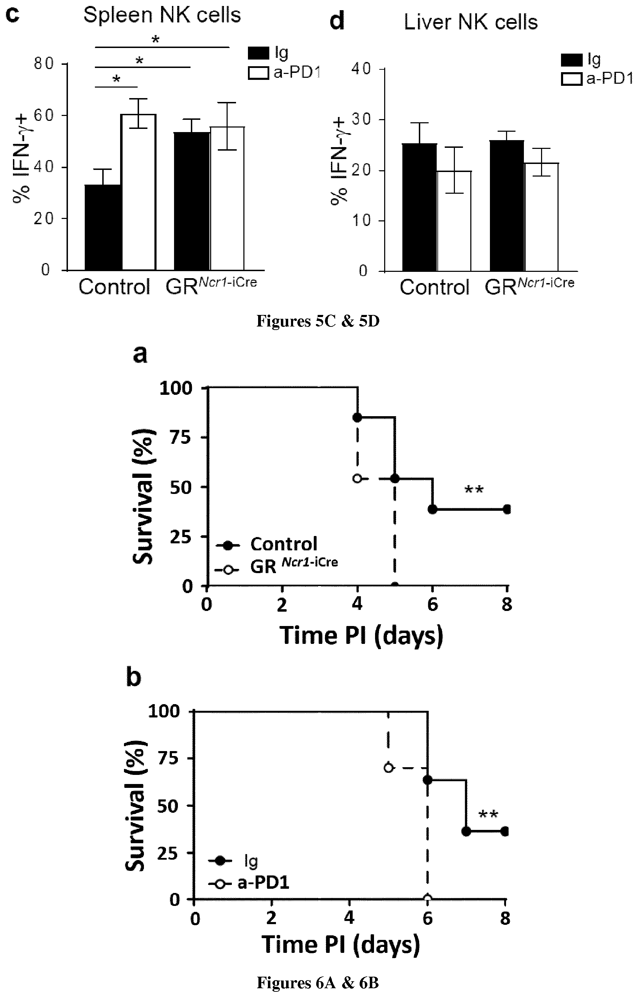

**Figure 5****: MCMV-induced IFN-γ production by spleen NK cells is regulated by a glucocorticoid-PD1 axis. a, b,** Representative FACS histograms of PDL1 **(a)** and PDL2 **(b)** expression 44 h PI in the spleen by macrophages (CD11c⁻CD11b⁺F480⁺), DCs (CD11c⁺MHCIIhi), neutrophils (CD11c⁻CD11b⁺Ly6G⁺), NK cells (NK1.1⁺NKp46⁺CD49b⁺), B cells (CD19⁺) and T cells (CD3⁺NK1.1⁻) (*n*=2-5, 2 experiments). **c, d,** Frequency of IFN-γ-producing NK cells in the spleen **(c)** and liver **(d)** 44 h PI, following treatment with an anti-PD1 antibody or an isotype control antibody (Ig) (*n*=6-9, pool of 4 experiments) (means ± SEM and representative FACS plots, **P*<0.05, one-way ANOVA).

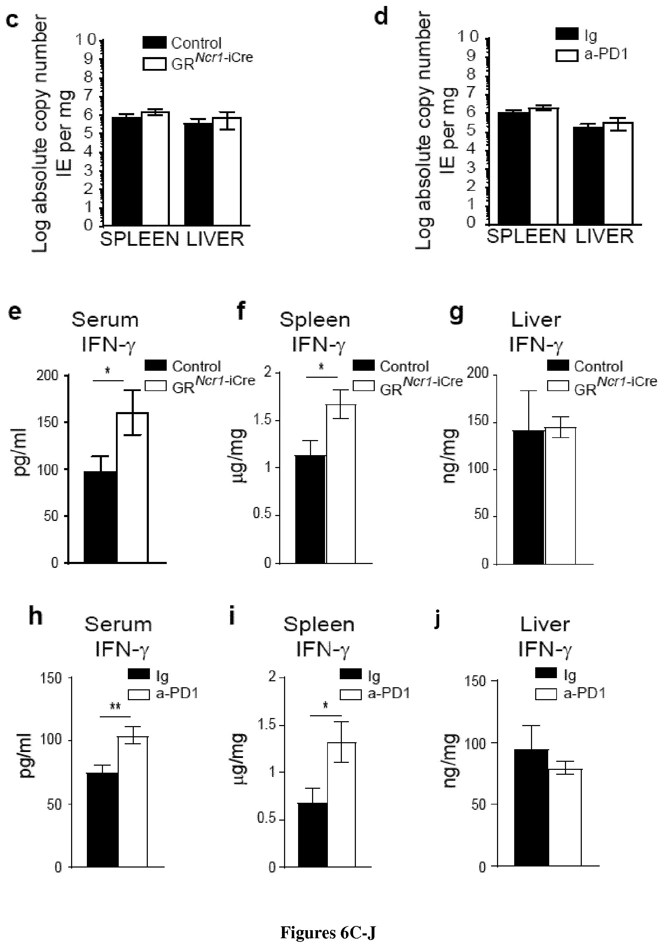

**Figure 6****: The glucocorticoid-PD1 regulatory pathway is required for protection against MCMV infection. (a)** Survival curve for mice infected with MCMV (*n*=13, pool of 3 experiments), and **(b)** receiving injections of anti-PD1 antibody or an isotype control antibody (Ig) (*n*=11-10, pool of 2 experiments) (***P*<0.01, log-rank Mantel-Cox test). **c** (*n*=6-9, pool of 2 experiments) and **d** (*n*=9-10, pool of 2 experiments), Viral titer 3 days PI. **e-j,** IFN-γ concentration 3 days PI **(e,** *n*=7-8, pool of 2 experiments, **P*<0.05, Mann-Whitney test) **(f, g** *n*=8-14, pool of 3 experiments, **P*<0.05, Student's t-test) **(h** *n*=7-8, pool of 2 experiments, ***P*<0.05, Mann-Whitney test) **(i, j,** *n*=9, pool of two experiments, **P*<0.05, Student's t-test). Means ± SEM.

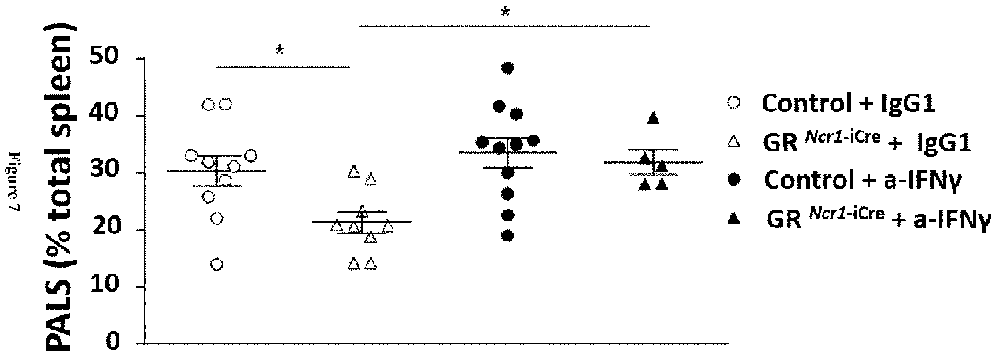

**Figure 7****: IFN-γ neutralization prevents spleen immunopathology in GR^{*Ncr1-i*Cre} mice.** Mice were infected with a high dose of MCMV and injected at day 1 PI with anti-IFNγ antibody or IgG1 isotype control. The area of periarteriolar lymphoid sheaths (PALS) was measured and the percentage over the total surface of the spleen section was calculated (*n*=5-11, pool of 2 experiments. Each symbol represents an individual mouse) (**P*<0.05, one-way ANOVA).

Example (not according to the invention as claimed but illustrating the mechanisms involved):

### Material & Methods

### Mice

C57BL/6J mice were purchased from Janvier Labs; GR^{*Ncr1-i*Cre} mice were generated as previously described, and *Ncr1*^{iCre/+} littermates were used as wt control. All the mice used were bred and maintained under specific pathogen-free conditions at the *Centre d'Immunophenomique (Ciphe) de Marseille* and the *Centre d'Immunologie de Marseille Luminy.* Mice were housed under a standard 12 h:12 h light-dark cycle with *ad libitum* access to food and water. Age-matched (7-10 weeks old) and sex-matched littermate mice were used as controls. All experiments were conducted in accordance with institutional committee recommendations and French and European guidelines for animal care.

### MCMV infection

Stocks of Smith strain MCMV were generated by homogenizing salivary glands harvested from six-week-old BALB/c mice infected with 2.5 x 10³ PFU of MCMV at the age of three weeks. Mice were infected at 2 p.m., by the ip injection of 10³ PFU/g or 3 x 10³ PFU/g (lethal dose) MCMV diluted in DMEM. "Non-infected" (NI) mice received DMEM only. For PD-1 blockade experiments, 250 ug anti-PD1 Ab (clone J43) or Armenian Hamster IgG (both from BioXCell) were injected i.p. into mice on day 1 post-infection. For IFN-gamma neutralization, 500 ug anti-IFNgamma Ab (clone XMG1.2) or Rat IgG1 (HRPN) (both from BioXCell) were injected i.p. into mice on day 1 post-infection. Spleens and livers were harvested after perfusion at different time points, and were processed for FACS or histology analysis, or weighed and homogenized for RNA or protein extraction. Organs were homogenized in a FastPrep-24 ^{™} 5G homogenizer (MP Biomedicals).

### Viral titer and RT-PCR

Organs were kept in RNAlater (Qiagen) after harvesting. RNA was extracted from organ homogenates with the RNeasy Fibrous Tissue Mini Kit (Qiagen), and reverse-transcribed with the iScript cDNA Synthesis kit (Biorad). Viral titers were determined, by qPCR, as absolute levels of the *Ie1* gene using the SYBR Green Master Mix (Takara). For *Il-15, Il-12* and *Il-18* gene expression analysis microfluidic RT-PCR with the Biomark HD system (Fluidigm) was used. Briefly, pre-amplified cDNA (22 cycles) was diluted fivefold before analysis in a Flex Six IFC (Fluidigm) with Universal PCR Master Mix (Fluidigm) and ready-to-use primer and probe sets pre-developed by Applied Biosystems (TaqMan Gene Expression Assays): IL-15 (Mm00434226_m1), IL-12b (Mm00434174_m1), IL-18 (Mm00434226_m1) and GAPDH as a housekeeper (Mm99999915_g1). Ct values were calculated from the system's software (BioMark Real-time PCR Analysis; Fluidigm).

### In vitro splenocytes stimulation

Splenocytes from control and GR^{*Ncr1-i*Cre} mice were stimulated *in vitro* in complete culture medium (RPMI 10% FCS, 100 µg/ml penicillin/streptomycin, 2 mM l-glutamine, 1 mM sodium pyruvate, and 0.01 M Hepes) with: 25 ng/ml IL-12 (eBiosciences), 25 ng/ml IL-15 (Peprotech) and 20 ng/ml IL-18 (MBL) alone or in combination, or with PMA (200ng/ml, Sigma) and ionomycin (1ug/ml, Sigma). For NK1.1 stimulation 96 well plates (Immulon 2HB) were coated overnight at 37°C with 2.5 ul/well anti-NK1.1 Ab (PK136, eBiosciences) or IgG2a isotype control before cell plating. During stimulation, 250 or 500 nM corticosterone (Sigma; dissolved in ethanol) or the same volume of vehicle alone were added to the medium. Cells were stimulated at 37°C in the presence of Golgi Stop and Golgi Plug from BD Biosciences. After 4 h of stimulation, the cells were washed and stained for FACS analysis. For CD107a staining, 2.5 ul/well of anti-CD107a-FITC antibody (1D4B, BD Biosciences) were added during stimulation.

### Cytokine levels in tissues

We determined IFN-gamma, IL-6, TNF and IL-10 protein levels in organ homogenates with cytometric bead arrays, according to the manufacturer's protocol (CBA, BD Biosciences). Results obtained in pg/ml were converted to mg/mg considering the weight of the organ before homogenization.

### Serum analysis

Blood was collected from the retro-orbital sinus of MCMV-infected mice under low-stress conditions (i.e., within 2 min of handling). After blood coagulation at room temperature, blood samples were centrifugated to separate the serum from the cloth. Serum samples were analyzed with the Corticosterone ELISA Kit (Enzo), according to the manufacturer's instructions, to determine corticosterone concentration, or were analysed with the cytometric bead arrays, according to the manufacturer's protocol (CBA, BD Biosciences), to determine cytokines concentration.

### Flow cytometry

Single-cell suspensions were obtained from the spleen by scratching it through 70um cell strainer, or from the liver by scratching it through 100um cell strainer and subsequent lymphocyte isolation on a 37.5%-67.5% Percoll gradient. For isolation of small intestine lamina propria cells, intestines were cut longitudinally, then transversally in 2-3cm pieces, thoroughly rinsed with PBS, and shaken for 30 minutes in PBS containing 10% FBS, 15mM Hepes and 5mM EDTA to remove intraepithelial and epithelial cells. Intestines were then digested with collagenase VIII (300UI/mL; Sigma) in complete RPMI for 45 minutes at 37°C under agitation, and lamina propria lymphocytes were isolated on a 40%-100% Percoll gradient. Cells were incubated with the Fc blocking antibody (2.4G2) and with a fixable blue dead-cell stain kit (Invitrogen). Surface molecules were stained with antibodies against: CD45.2 (104), CD3 (145-2C11), CD19 (1D3), NK1.1 (PK136), CD49a (Ha31/8), CD11b (M1/70), MHCII (M5/114.15.2), TCRb (H57-597), PD1 (J43, and Hamster IgG2 isotype control), PDL1 (MIH5) from BD Biosciences; NKp46 (29A1.4), CD49b (DX5), F4/80 (BM8), Ly49H (3D10) from eBioscience; CD11c (N418), PD1 (RMP1-30, and Rat IgG2b isotype control), PDL2 (TY25) and Ly6G (1A8) from Biolegend. For intracellular staining, cells were fixed and permeabilized with an intracellular staining kit (eBioscience), and the following antibodies were used: anti-IFN- (XMG1.2) from Biolegend; anti-granzyme B (GB11), anti-Rort (Q31-378), anti-IL17 (TC11-18H10), anti-Ki67 (B56) from BD Biosciences, anti-IL-22 (JOP eBioscience, coupled to Alexa Fluor 647 with an antibody labeling kit from Life Technologies), anti-GR XP rabbit mAb (D8H2) and rabbit mAb IgG XP (DA1E) from Cell Signaling Technology. Stained samples were analyzed in a BD LSRII flow cytometer (BD Biosciences). Apoptosis was detected with the FITC Annexin V Apoptosis Detection Kit I from BD Biosciences, according to the manufacturer's protocol.

### Histology

Tissues were fixed in 10% neutral buffered formalin for 24h, dehydrated and embedded in paraffin. Sections of 3.5 um were cut using the microtome Leica RM2245. Hematoxylin-eosin (H&E) staining was effectuated automatically with Leica autostainer XL and slides were mounted with entellan and kept at room temperature. Histological slides of spleen and liver tissue were assessed by an anatomopathologist in a blinded way. For spleen inflammation grading, a score was assigned based on the severity: 0 for normal spleen, 1 for mild (multifocal pyogranulomas in marginal zones), 2 for moderate (locally coalescing pyogranulomas in marginal zones with small necrotic foci), 3 for marked (large and coalescing pyogranulomas throughout the splenic parenchyma with extensive necrotic foci, PALS are preserved), and 4 for severe (extensive necrotic and pyogranulomatous foci, PALS are partially replaced by necrotic and granulomatous inflammation). For liver inflammation grading, a score was assigned based on the severity: 0 for normal, 1 for mild (multifocal pyogranulomatous hepatitis with scattered single necrotic hepatocytes), 2 for moderate (multifocal to coalescing necrotic and pyogranulomatous hepatitis with intranuclear inclusions in hepatocytes), and 3 for marked (coalescing necrotic and pyogranulomatous hepatitis with intranuclear inclusions in hepatocytes). For the morphometric assessment of periarteriolar lymphoid sheath (PALS), the area of PALS and the total area of spleen section were measured using the ImageJ software. Analysis was done on pictures taken with Nikon Eclipse, on random cross sections of the spleens.

### Cell sorting and mRNAseq analysis

Splenocytes (after NK cell enrichment with the mouse NK Cell Isolation Kit II, Miltenyi Biotec) and liver lymphocytes were pooled from three mice for each genotype. A FACS Aria III (BD Biosciences) was used to sort approximately 5 x 10⁵ NK cells from the spleen and liver and 5 x 10⁴ liver-resident ILC1s. Cells were sorted directly in RLT lysis buffer (Qiagen). Total RNA was prepared from purified ILC populations with an RNeasy Micro Kit (Qiagen). Three biological replicates were generated for all samples except for the GR^{*Ncr1*-iCre} liver ILC1s sample (two biological replicates). Preamplification was performed with the SMART-Seq^{®} v4 Ultra^{®} Low-Input RNA Kit (Clontech). The DNA libraries were generated by double-indexing with the Nextera XT DNA Kit (Illumina) and RNA sequencing was performed with a NextSeq 500 (Illumina; paired-end reads 2x75 with 30 M reads per sample). The fastq files were assessed with the fastqc program and trimming was performed with Trimmomatics, to remove potential molecular barcodes, Illumina adapters and low-quality reads. Alignment was performed with two algorithms: firstly, with bowtie2 vs GRCm38 ensemble transcriptome resulting in a BAM that could be processed with the molecular index provided by the kit vendor, and, secondly, with HiSat2 over the GRCm38 genome, for the detection of novel junction regions. Duplicates were detected and removed by MarkDuplicates from picard tools, and the number of reads mapped to each gene was determined with featureCounts v1.5.2. Normalization and differential analysis were performed with DESeq2 v1.16.1. HalioDx (Marseille, France) processed the RNA samples and bioinformatics analyses were performed by the CIML platform.

### Statistical analysis

No sample size calculation was performed, but a reasonable sample size was chosen to ensure adequate reproducibility of results and was based on our previous studies. Mice were assigned to experimental groups according to sex and age. Statistical analysis was performed with Graphpad Prism 7 Software. Normality was tested with the Shapiro-Wilk test. Unpaired two-tailed Student's t-tests were used if the data followed a Gaussian distribution with similar variances. Mann-Whitney U tests were performed if this was not the case. One-way ANOVA was used for multigroup comparisons. Differences in survival were evaluated with Mantel-Cox tests. Differences were considered significant for P values less than 0.05.

### Results

### Endogenous glucocorticoids regulate IFN-gamma production by NCR1+ ILCs in an organ-specific manner

In agreement with previous studies, infection with MCMV induced the release of corticosterone in the blood circulation with a peak at 36h post-infection (Fig. 1a) suggesting that the activation of the HPA axis may play a role during the early phase of the infection when NK cells are activated. NK cells control MCMV infection through cytokine (IFN-gamma) production and killing-dependent mechanisms. The role of glucocorticoids on NK cell cytotoxic activity was first analyzed *in vitro.* Corsticosterone treatment did not affect their ability to degranulate (measured by their CD107a expression) upon stimulation with anti-NK1.1 antibodies or PMA and ionomycine (data not shown). GR^{*Ncr1-i*Cre} and control mice were then infected with MCMV, and we monitored their immune response after the corticosterone peak in the blood (44h post-infection, Fig. 1a). Upon infection, Granzyme B (GrzB), a surrogate marker of NK cell cytotoxicity, was up-regulated in NK cells (data not shown). However, GR-deficient and sufficient NK cells expressed similar levels of GrzB suggesting that their cytotoxic response is unaffected by endogenous glucocorticoid levels (data not shown). In contrast, in the spleen, the absence of GR expression in NK cells led to an increase in their IFN-γ production both at the per cell levels (mean fluorescence intensity) and in term of percentages of producing cells (Fig. 1b and data not shown). Corticosterone is produced systemically, we were thus surprised to observe that this regulation of IFN-gamma production by endogenous glucocorticoids was not observed in NK cell and ILC1s from the liver which are also known to express the GR (Fig. 1c, d). These data thus showed that the control of NCR1+ ILCs function by glucocorticoids is tissue-specific.

Given that NCR1+ ILC3s in GR^{*Ncr1*-iCre} mice also express GR, we assessed a possible modification of their function by endogenous glucocorticoids upon MCMV infection. These ILC3s are mainly resident in the small intestine and are IL-22 and IL-17 but not IFN-gamma producers. As such and in line with the higher virus tropism for the spleen and the liver, they were not described as playing a role in MCMV anti-viral response. However, we analyzed a possible modification of their function in GR^{*Ncr1-*iCre} infected mice. MCMV infection did not induce any increase of IL-22 and IL-17 production by NCR1+ ILC3 in control mice (data not shown). Analysing NCR1+ ILC3s from the small intestine of MCMV infected GR^{*Ncr1-i*Cre} mice, we didn't observed any difference in in term of cytokine production or cell frequency compared to control animals (data not shown). These data suggest that NCR1+ILC3s and their responsiveness to glucocorticoids do not have a major impact on the immune response to MCMV infection. We thus further analyzed the anti-viral immune response occurring in the spleen and liver of GR^{*Ncr1*-iCre} mice.

Consistent with the increased production of IFN-gamma by GR-deficient spleen NK cells (Fig. 1b), histological analysis showed that the extent of splenic lesions differed between control and GR^{*Ncr1-i*Cre} mice, with the mutant mice having more marked lesions, with coalescing and granulomatous necrotic splenitis in the marginal zone (Fig. 2a). By contrast, no significant difference in the nature or extent of liver lesions was observed, with mild multifocal pyogranulomatous and necrotic hepatitis in both types of mice (Fig. 2b). Remarkably, the higher level of inflammation observed in the spleen of GR^{*Ncr1*-iCre} mice was not due to a higher viral load (Fig. 2c), or to differences in corticosterone concentration in the serum (data not shown). Therefore, the organ-specific control of NK cell IFN-gamma production functions by endogenous corticosterone does not impair viral clearance but is associated a reduction of inflammation-induced tissue damage.

### Whole genome transcriptomic analysis of GR-regulated genes

We then aimed to dissect the intrinsic genome-wide mechanisms through which endogenous glucocorticoids regulate the function of the NCR1+ ILCs *in vivo,* in the main organs of viral replication. Spleen and liver NK cells and liver ILC1s from MCMV infected GR^{*Ncr1-i*Cre} and control mice were isolated by cell sorting and analyzed by RNA-sequencing (RNAseq). The pairwise comparison of gene expression between GR-sufficient and GR-deficient cells allowed to identify the genes regulated by the GR pathway triggered in the physiopathological context of a viral infection.

Remarkably, this unbiased genome-wide analysis revealed that the GR-dependent transcriptomic changes were different in the three NCR1⁺ ILC subsets analyzed (spleen and liver NK cells and liver ILC1s), indicating that the *in vivo* regulation of gene expression by glucocorticoids was both organ- and cell lineage-specific. For identification of the candidate genes responsible for the selective hyperinflammation observed in the spleen of GR^{*Ncr1*-iCre} mice (Fig. 2a), we focused on the DEGs between splenic NCR1⁺ cells (i.e. NK cells) from GR^{*Ncr1*-iCre} and control mice (Fig. 3a). Besides the GR-encoding gene *Nr3c1,* we found that the expression of *Tsc22d3* was significantly downregulated in both spleen and liver NK cells from GR^{*Ncr1*-iCre} mice relative to infected control mice. This result is consistent with the rapid and ubiquitous induction of Tsc22d3 by glucocorticoids described in many cell types and in many inflammatory conditions. *Tsc22d3* encodes for Glucocorticoid-induced leucine zipper (GILZ), recognized as an important mediator of GC anti-inflammatory effects, as it regulates survival, homeostasis and apoptosis in various cell types, including lymphoid cells. Despite the differential expression of this gene, no difference in the proliferation and apoptosis of splenic and liver NK cells was observed between GR^{*Ncr1*-iCre} mice and control littermates (data not shown). The homeostasis of liver ILC1s was also similar in the two strains of mice (data not shown). These data suggest that GILZ does not play a major role in the selective immunopathology observed in the spleen of GR^{*Ncr1*-iCre} mice.

### Endogenous glucocorticoids induce PD1 expression on spleen NK cells

The only gene found to be differentially regulated by the GR in spleen NK cells but not in liver NK cells or liver ILC1s (Fig. 3a) was *Pdcd1.* This gene encodes the immune checkpoint PD1 (programmed cell death protein 1), an inhibitory cell-surface receptor that downregulates T-cell activity. We analyzed the expression of the PD1 protein on immune cells upon MCMV infection. PD1 was not detected at surface of NK cells from uninfected mice (Fig. 3b). By contrast, MCMV infection induced expression of the PD1 protein on spleen NK cells (Fig. 3b). Importantly, this induction was strictly dependent on the glucocorticoid-GR pathway, as PD1 was not upregulated in NK cells from infected GR^{*Ncr1*-iCre} mice (Fig. 3b). At this time point after infection (44h), PD1 expression was not detected on spleen macrophages, neutrophils or DCs (data not shown). At steady state a discrete subset of T cells (less than 4%) expressed a low basal level of PD1, but this subset was not expanded and its PD1 expression was unchanged upon infection (Fig. 3c). Remarkably, consistent with the transcriptomic data, glucocorticoid-induced PD1 expression on NK cells was tissue-specific, as it was not observed on the surface of NK cells and ILCls in the liver (Fig. 3d, e). Of note, the same results were obtained using two different clones of anti-PD1 antibodies (J43 and RMP1-30).

The specific induction of PD1 by glucocorticoids on spleen but not liver NK cells could not be explained by the lack of expression of GR in liver NK cells at steady state or upon MCMV infection. Along this line, the transcriptomic data showed that liver NK cells were responsive to the GR signalling as the *Tsc22d3* gene expression was similarly modified in spleen and liver NK cells of GR^{*Ncr1*-iCre} mice. We thus hypothesized that the regulation of PD1 expression by the GR could be dependent on the specific combination of inflammatory cytokines present in the tissue-microenvironment. We measured by qRT-PCR the expression of IL-12, IL-15 and IL-18, the main cytokines known to modulate NK cell activation. Although we didn't find a significant induction of IL-18 44h post- MCMV infection either in the spleen or in the liver (data not shown), we found that IL-12 expression was mainly induced in the liver while IL-15 was only induced in the spleen but not in the liver (Fig. 4a). This result shows that upon MCMV infection the cytokine micro-environment is different between the two organs raising the question of a potential impact on the GR regulation. To further dissect how a different combination of cytokines in the milieu could affect the role of the GR on NK cells, we performed *in vitro* experiments. Splenocytes were stimulated with different combinations of the cytokines IL-12, IL-15 and IL-18 in the presence or absence of corticosterone (Fig. 4b). Corticosterone alone or in the presence of IL-15 was not able to induce PD1 expression (data not shown and Fig. 4b). Only the simultaneous incubation of corticosterone to the combination IL-15 + IL-18 was able to induce PD1 expression on NK cells, in a dose-dependent manner (Fig. 4b). Unexpectedly, addition of IL-12 was instead capable to abrogate this effect. Of note, the PD1 induction by corticosterone in addition to IL-15 and IL-18 was not observed on T cells or NK cells from GR^{*Ncr1-i*Cre} mice, demonstrating that it is a cell specific cell intrinsic effect dependent on GR (Fig. 4b). These results show that the final outcome of GR signaling is dependent on the simultaneous stimulation by inflammatory cytokines and suggest that the lack of expression of PD1 in liver NK cells is due to the absence of production of IL-15 associated with higher levels of IL-12.

### A GR-PD1 axis inhibits IFN-gamma production by spleen NK cells in infected mice

We further investigated the functional relevance of PD1 expression on spleen NK cells in this infectious model, by determining whether the ligands of this molecule, PD-L1 and PD-L2, were also induced during MCMV infection. We found that PD-L1 was strongly induced on spleen macrophages, DCs, neutrophils, T, B and NK cells, and PD-L2 was expressed on DCs (Fig. 5a, b). Overall, these data suggest that the PD1 inhibitory pathway may be involved in the control of NK cell function in the spleen of GR-sufficient hosts.

In C57BL/6 mice, the activating NK cell receptor Ly49H mediates resistance to MCMV infection due to the specific binding of m157, a virally encoded protein. We investigated if there was a correlation between PD1 and Ly49H expression on NK cells, and we found that PD1 was upregulated on both Ly49H+ and Ly49H- NK subsets 44h post-infection (data not shown). Moreover, control and GR^{*Ncr1-i*Cre} mice displayed a comparable expansion of Ly49H+ subset 5 days post-infection (data not shown), suggesting that GR signaling and the consequent PD1 expression doesn't contribute to the expansion of antigen-specific NK cells in MCMV infection.

To verify whether the PD1 inhibitory pathway could be involved in the control of NK cell activation in the spleen, mice were infected with MCMV in the presence or absence of anti-PD1 antibodies that block the interaction of PD1 with its ligands. Anti-PD1 and isotype control antibodies were injected at day 1 post-infection, just before HPA axis activation and glucocorticoid systemic release. At 44 h post-infection, PD1 blockade increased the frequency of IFN-gamma⁺ NK cells in the spleen of control mice to levels similar to those observed in GR^{*Ncr1*-iCre} mice (Fig. 5c). This enhancement of IFN-gamma production in spleen NK cells did not occur in antibody-treated GR^{*Ncr1-i*Cre} mice, demonstrating a strict dependence of the effect of PD1 on GR expression in NCR1⁺ cells. Moreover, consistent with the lack of PD1 expression on NK cells in the liver (Fig. 3d), we observed no effect of PD1 blockade on IFN-gamma production in these cells (Fig. 5d). Thus, during MCMV infection, endogenous glucocorticoids induce PD1 expression on NK cells, which, in turn, leads to the control of IFN-gamma production by these cells. This regulation depends on tissue microenvironment, as it occurs in the spleen but not in the liver.

### Glucocorticoid-induced PD1 expression on NK cells is required for resistance to MCMV infection

We then assessed the impact of this glucocorticoid-PD1 axis on host resistance to viral infection, by infecting mice with a lethal dose of MCMV. Mortality was higher for GR^{*Ncr1*-iCre} mice than for their control littermates (Fig. 6a), demonstrating the requirement for glucocorticoid signaling in NCR1⁺ ILCs for host resistance to viral infection. We evaluated the specific contribution of PD1-PD-L interaction to the higher survival rate of GR-sufficient mice, by infecting wild-type (wt) mice with and without PD1 blockade. All mice receiving injections of anti-PD1 antibodies succumbed to the infection within the first week (Fig. 6b), resulting in a mortality rate similar to that for GR^{*Ncr1-i*Cre} mice. This greater susceptibility of infection was not due to differential viral replication in GR^{*Ncr1-i*Cre} mice (Fig. 6c) or upon anti-PD1 treatment in wt mice (Fig. 6d), indicating that the GR regulatory pathway does not impair viral clearance. Like infected GR^{*Ncr1-i*Cre} mice (Fig. 2a), wt mice receiving anti-PD1 antibodies presented more marked features of spleen immunopathology (data not shown). In particular, they displayed larger areas of coalescing necrotic and granulomatous splenitis in the marginal zone than mice receiving control Ig injections (data not shown). Similar results were obtained with two different clones of anti-PD1 antibodies (J43 and RMP1-14). By contrast, hepatic lesion severity was not affected by PD1 blockade, with both mice displaying moderate to marked necrotic and pyogranulomatous hepatitis with intranuclear inclusions in hepatocytes (data not shown). PD1 blockade and specific GR depletion in NCR1⁺ ILCs were both associated with a higher systemic concentration of IFN-gamma and an increased IFN-gamma production in the spleen, but not in the liver of infected mice (Fig. 6e-1). Moreover, among the MCMV-induced cytokines measured in GR^{*Ncr1*-iCre} and in anti-PD1 treated mice IFN-gamma was the only one to be increased, while the production of IL-6, TNF-alpha and IL-10, was unaffected (data not shown). Wt mice treated with anti-PD-1 are, therefore, phenocopies of GR^{*Ncr1*-iCre} mice. These data demonstrate an essential role for a neuroendocrine-immune pathway involving glucocorticoids and PD1 in promoting host protection against MCMV infection without affecting viral replication.

### Increased IFN-gamma in GR^{Ncr1-iCre} mice determines spleen immunopathology

IFN-gamma is required for anti-viral response to MCMV. While its systemic depletion impairs virus elimination and increases mortality, over-production of IFN-gamma doesn't affect viral clearance but, on the contrary, is associated to increased mice susceptibility to the infection (Fig. 6). We thus hypothesized that the lack of control of NK cells IFN-gamma production by glucocorticoids in the spleen of GR^{*Ncr1-i*Cre} mice could lead to hyperinflammation and immunopathology.

At day 3 post-high dose MCMV infection, we observed marked to severe inflammation in the spleen, with large and coalescing pyogranulomas and necrotic foci throughout the splenic parenchyma (data not shown). We observed a destruction of the white pulp, in particular of the periarteriolar lymphoid sheath (PALS), that displayed decreased cellularity and atrophy in the severely affected areas of the spleen. We found that in the spleens of GR^{*Ncr1-i*Cre} mice PALS were partially replaced by necrotic and granulomatous inflammation. To quantify the severity of the splenic lesions at this time of the infection and with this dose of virus we performed a morphometric assessment of PALS measuring the percentage of PALS area over total splenic section. We found that GR^{*Ncr1*-iCre} mice displayed a more severe destruction of splenic architecture, with a decreased surface of PALS on total splenic section area compared to control mice (Fig. 7). To verify whether this phenotype was dependent on IFN-gamma, we injected anti-IFN-gamma neutralizing antibody at day 1 PI, concomitantly with the activation of the HPA axis, to counteract uncontrolled production of IFN-gamma by GR-deficient NK cells in the spleen. We found that IFN-gamma neutralization didn't significantly affect the severity of splenic lesions in Control mice, while it rescued the immunopathology in GR^{*Ncr1*-iCre} mice to a level similar to the Control (Fig. 7).

Taken all together these data show that a GR-PD1-IFNgamma axis is required in NK cells to protect the host upon MCMV infection, by preventing spleen hyperinflammation and IFNgamma-mediated immunopathology.

## Claims

1. An antibody drug conjugate comprising an antibody that binds to a specific marker of innate lymphoid cell conjugated to an agonist of glucocorticoid receptor for use in a method of treating an infectious disease, wherein:
- the innate lymphoid cell is natural killer cell
- the specific marker of innate lymphoid cell is NCR1 and
- the infectious disease is caused by a virus.

## Patentansprüche

1. Antikörper-Wirkstoff-Konjugat, welches einen Antikörper umfasst, welcher an einen spezifischen Marker einer angeborenen Lymphoidzelle bindet, welcher an einen Agonisten des Glukokortikoidrezeptors konjugiert ist, zur Verwendung in einem Verfahren zum Behandeln einer Infektionskrankheit, wobei:
- die angeborene Lymphoidzelle eine natürliche Killerzelle ist
- der spezifische Marker der angeborenen Lymphoidzelle NCR1 ist und
- die Infektionskrankheit durch ein Virus verursacht wird.

## Revendications

1. Conjugué anticorps-médicament comprenant un anticorps qui se lie à un marqueur spécifique de cellule lymphoïde innée conjugué à un agoniste du récepteur des glucocorticoïdes, destiné à être utilisé dans un procédé de traitement d'une maladie infectieuse, dans lequel :
- la cellule lymphoïde innée est une cellule tueuse naturelle
- le marqueur spécifique de cellule lymphoïde innée est NCR1 et
- la maladie infectieuse est provoquée par un virus.
